# FASCICULE DE BREVET EUROPEEN

(11) **EP 1 773 829 B1**
(45) Date de publication et mention de la délivrance du brevet: **17.12.2008**
(21) Numéro de dépôt: 05790816.2
(22) Date de dépôt: 20.07.2005
(51) Int. Cl.: C07D 451/04, A61K 31/46, A61K 31/55, A61P 3/04, A61P 3/10, A61P 15/00

(54) **DERIVES D'AMINO-TROPANE, LEUR PREPARATION ET LEURS APPLICATIONS EN THERAPEUTIQUE**
AMINOTROPANDERIVATE, DEREN HERSTELLUNG UND DEREN THERAPEUTISCHE VERWENDUNG
AMINO-TROPANE DERIVATIVES, PREPARATION THEREOF AND THERAPEUTIC USE THEREOF

(30) Priorité: 29.07.2004 FR 0408372
(43) Date de publication de la demande: 18.04.2007
(73) Titulaire: Sanofi-Aventis, 75013 Paris (FR)
(72) Inventeur: BRAUN, Alain, F-92100 Boulogne Billancourt (FR); CORNET, Bruno, F-77930 PERTHES-EN-GATINAIS (FR); COURTEMANCHE, Gilles, F-31120 Lacroix-Falgarde (FR); CRESPIN, Olivier, F-95000 Cergy (FR); PASCAL, Cécile, F-92500 Rueil-Malmaison (FR)
(74) Mandataire: Morel-Pécheux, Muriel
(86) Numéro de dépôt international: PCT/FR2005/001856
(87) Numéro de publication internationale: WO 2006/021657

(56) Documents cités:
- WO-A-02/059095
- WO-A-03/061660
- IRANI, B.G. ET AL.: "Progress in the Development of Melanocortin Receptor Selective Ligands" CURRENT PHARMACEUTICAL DESIGN, vol. 10, 2004, pages 3443-3479, XP001205465
- HOLDER, J.R.; HASKELL-LUEVANO,C.: "Melanocortin Ligans: 30 Years of Structures-Activity Relationship" MEDICINAL RESEARCH REVIEWS, vol. 24, no. 3, 2004, pages 325-356, XP009044667

## Description

La présente invention se rapporte à des composés agonistes des récepteurs aux mélanocortines, à leur préparation et à leur application en thérapeutique.

Les récepteurs aux mélanocortines (MC-Rs) appartiennent à la super-famille des récepteurs couplés aux protéines G présentant sept domaines transmembranaires. Leur voie de transduction passe par la production d'AMPc (Cone, R. D., Recent Prog. Horm. Res., 1996, 51, 287*).* Cinq sous types de MC-Rs sont actuellement décrits, MC1-R, MC2-R, MC3-R, MC4-R et MC5-R, et sont exprimés dans différents tissus tels que le cerveau (MC3, 4, 5-R), les glandes exocrines (MC5-R), les surrénales (MC2-R) et la peau (MC1-R) pour les principaux. Les ligands naturels des MC-Rs sont, pour les agonistes, l'ACTH, l'α, β et γ-MSH, et pour les antagonistes, l'agouti protéine et l'agoutirelated protéine. Aucun des ligands naturels n'est très sélectif pour l'un des sous-types, à l'exception du γ-MSH, qui possède une certaine sélectivité pour le MC3-R.

Le système mélanocortine est impliqué dans de nombreux processus physiologiques, incluant la pigmentation, l'inflammation, le comportement alimentaire et sexuel (notamment la fonction érectile), la balance énergétique (régulation du poids corporel et stockage lipidique), les fonctions exocrines, la protection et régénération neuronale, l'immunomodulation, l'analgésie, etc ...

Notamment, il a été démontré que le MC4-R est impliqué dans le comportement sexuel (Van der Ploeg, L. H., Proc. Natl. Acad. Sci. USA, 2002, 99, 11381 *;* Martin, W. J., Eur. J. Pharmacol., 2002, 454, 71). Il a également été démontré, par l'intermédiaire de modèles de souris spécifiquement dépourvues en certains MC-Rs (souris knockout), que les MC-Rs centraux (MC3 et 4-R) étaient impliqués dans le comportement alimentaire, l'obésité, le métabolisme et la balance énergétique *(*Huszar, D., Cell, 1997, 88(1), 131 *;* Chen, A. S., Nat. Genet., 2000, 26(1), 97; Butler, A.A., Trends Genet., 2001, 17, S50-S54). Ainsi, les souris knockout MC4-R sont hyperphagiques et obèses. Parallèlement, les antagonistes aux MC3 et/ou 4R favorisent la prise de nourriture, tandis que la stimulation des MC4-R par un agoniste endogène, tel que l'α-MSH, produit un signal de satiété.

Ces observations laissent penser que la stimulation du MC3-R et/ou du MC4-R central, en réduisant la prise de nourriture et le poids corporel, est une approche prometteuse pour traiter l'obésité, risque aggravant de nombreuses autres pathologies (hypertension, diabète, ...). Ainsi, les recherches ont permis d'identifier dans un premier temps des peptides, pseudo-peptides ou peptides cycliques, capables d'interagir avec les MC-Rs et de moduler ainsi la prise de nourriture.

Afin de maintenir sur le long terme une perte de poids efficace et limiter ainsi les, co-morbidités, un traitement quotidien à long terme doit être envisagé. Ceci implique qu'un médicament, pour cette indication thérapeutique, doit pouvoir être administré par une voie simple pour le patient. La voie orale doit donc être privilégiée. Or, les composés peptidiques ne sont généralement pas les plus appropriés pour répondre à cette obligation. C'est pourquoi il est important de développer des petites molécules non peptidiques.

Dans cette optique, les demandes internationales PCT publiées sous les numéros WO 02/059095, WO 02/059108, WO 03/009850 et WO 03/061660 décrivent des dérivés de type pipérazine. D'autres demandes décrivent des dérivés de type pipéridine, telles que les WO 03/092690 et WO 03/093234. Les demandes WO 99/64002 et WO 01/70337 décrivent des dérivés de type spiro-pipéridine. La demande WO 01/91752 décrit des dérivés comportant un motif pipéridine fusionné avec un noyau pyrazolyle. La demande WO 02/059107 décrit des dérivés de type pipéridine et pipérazine substitués par une structure bicyclique. Les demandes WO 02/059117, WO 02/068388 et WO 03/009847 décrivent des dérivés de type pipéridine et/ou pipérazine substitués par un noyau phényle. Quant à la demande WO 03/094918, elle décrit des dérivés de type pipérazine substitués par un noyau phényle ou pyridinyle. On peut également citer les demandes WO 00/74679, WO 01/70708, WO 02/15909, WO 02/079146, WO 03/007949 et WO 04/024720, qui décrivent des dérivés de type pipéridine substituée, ou encore la demande WO 04/037797 ; les composés décrits dans ces demandes de brevets comportent toujours une fonction amide, mimant les structures peptidiques antérieurement connues.

Face au besoin constant d'améliorer les thérapies existantes pour les pathologies évoquées précédemment, les Inventeurs se sont donnés pour but de pourvoir à de nouveaux composés agonistes des récepteurs aux mélanocortines. La présente invention a pour objet des composés répondant à la formule (I) 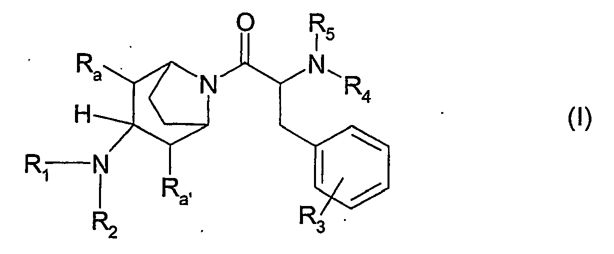 dans laquelle :
**Rₐ** et **Rₐ**^{,}, identiques ou différents l'un de l'autre, représentent un atome d'hydrogène ou un groupe alkyle ou cycloalkyle,
**R₁** représente un atome d'hydrogène ou un groupe alkyle, cycloalkyle, hétérocycloalkyle ou aryle,
**R₂** représente un groupe de formule -(CH₂)ₓ(CO)y-Y ou -(CO)_{y}-(CH₂)ₓ-Y, dans laquelle :
   - x=0, 1, 2, 3 ou 4,
   - y= 0 ou 1,
   - Y représente un atome d'hydrogène ou un groupe hydroxyle, alkyle, cycloalkyle, alcoxy, aryle, hétéroaryle ou -NR₁₁R₁₂, Y étant différent d'un atome d'hydrogène lorsque x=y=0,
   - R₁₁ et R₁₂, identiques ou différents l'un de l'autre, représentent un atome d'hydrogène ou un groupe alkyle, cycloalkyle, alcoxy ou -NR₁₃R₁₄, ou bien R₁₁ et R₁₂ forment ensemble, avec l'atome d'azote auquel ils sont rattachés, une structure mono-ou bicyclique comportant de 4 à 10 chaînons et comprenant éventuellement 1 à 3 hétéroatomes supplémentaires et/ou 1 à 3 insaturations éthyléniques ou acétyléniques, ce cycle étant éventuellement substitué en des positions quelconques par 1 à 3 groupes choisis parmi les atomes d'halogène et les groupes hydroxyles, alkyles, cycloalkyles et alcoxy. A titre d'exemples de telles structures cycliques, on peut citer les groupes pyrrolidinyle, morpholinyle, pyrrolinyle, isoindolinyle, etc ...,
   - R₁₃ et R₁₄, identiques ou différents l'un de l'autre, représentent un atome d'hydrogène ou un groupe alkyle, cycloalkyle ou alcoxy, ou bien R₁₃ et R₁₄ forment ensemble, avec l'atome d'azote auquel ils sont rattachés, une structure mono- ou bicyclique telle que définie ci-dessus,
**R₃** représente 1 à 3 groupes, identiques ou différents les uns des autres, situés en des positions quelconques du noyau auquel ils sont rattachés et choisis parmi les atomes d'halogène et les groupes alkyles, cycloalkyles, -OR, -NRR', -CO-NRR', -NR-COR', -NR-CO-NRR', -NR-COOR', -NO₂, -CN et -COOR, où R et R' sont tels que définis ci-dessous,
**R₅** représente un atome d'hydrogène ou un groupe alkyle ou cycloalkyle,
**R₄** est choisi parmi :
   (1) un groupe de formule (a), (b) ou (c) éventuellement substitué par un groupe oxo: 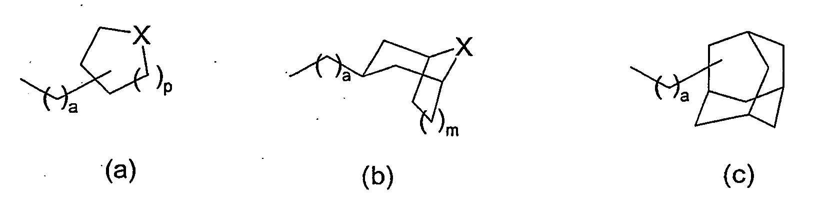 dans lesquelles chacun des cycles de formules (a), (b) et (c) peut être substitué, en des positions quelconques, par 1 à 4 groupes R₇, identiques ou différents les uns des autres, et dans lesquelles :
      **a** = 0, 1, 2 ou 3,
      **p** = 0, 1, 2 ou 3,
      **m** = 0, 1 ou 2,
      **X** représente un atome d'oxygène ou de soufre ou un chaînon -C(R₆)(R₇)- ou -N(R₁₀)-,
   **R₆** est choisi parmi :
   - un atome d'hydrogène, un atome d'halogène
   - un groupe -(CH₂)ₓ OR₈, -(CH₂)ₓCOOR₈, -(CH₂)ₓ NR₈R₉, -(CH₂)ₓ-CO-NR₈R₉ ou -(CH2)ₓ-NR₈-COR₉, dans lesquels x = 0, 1, 2, 3 ou 4 et R₈ et R₉ sont tels que définis ci-dessous,
   - un groupe alkyle, cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle ou -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉,
   - un groupe cycloalkyle ou hétérocycloalkyle fusionné ou non situé en position spiro sur le cycle de formule (a) auquel il est rattaché,
   - un groupe cycloalkyle ou hétérocycloalkyle fusionné avec un groupe aryle ou hétéroaryle,
   **R₇** est choisi parmi les atomes d'hydrogène et d'halogène et les groupes alkyles, cycloalkyles, aryles, hétéroaryles, alkylaryles, alkylhétéroaryles, -OR, -O-aryles, -O-hétéroaryles, -O-alkylaryles, -O-alkylhétéroaryles, -NRR', -CO-NRR', -NR-CO-R', -NR-CO-NRR', -NR-COOR', -NO₂, -CN et -COOR, où R et R' sont tels que définis ci-dessous,
   **R**₈ et **R₉** sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles -SO₂-cycloalkyles, -SO₂-hétérocycloalkyles, -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4 et R et R' sont tels que définis ci-dessous, les groupes alkyles, cycloalkyles, hétérocycloalkyles, aryles et hétéroaryles étant éventuellement substitués par un ou plusieurs groupes choisis parmi les les groupes R, R', -OR, -NRR', -CO-NRR', -NR-CO-R', -NR-CO-NRR', -NO₂, -CN et -COOR, OCOR, COR, OCONRR', NRCOOR'
   ou bien R₈ et R₉ forment ensemble un cycloalkyle ou un hétérocycloalkyle ;
   **R₁₀** est choisi parmi :
   - un atome d'hydrogène,
   - un groupe -(CH₂)ₓ-OR₈, -(CH₂)ₓ-COOR₈, -(CH₂)ₓ-NR₈R₉, -(CH₂)ₓCO-NR₈R₉ ou -(CH₂)ₓ-NR₈-COR₉, dans lesquels x = 0, 1, 2, 3 ou 4 et R₈ et R₉ sont tels que définis ci-dessus,
   - un groupe cycloalkyle ou hétérocycloalkyle fusionné avec un groupe aryle ou hétéroaryle,
   - un groupe alkyle, cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle, -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉, -SO₂-alkyle, -SO₂-cycloalkyle, -SO₂-hétérocycloalkyle, -SO₂-aryle, -SO₂-hétéroaryle, -SO₂-alkylaryle, -SO₂-alkylhétéroaryle ou -SO₂-NR₈R₉, où R₈ et R₉ sont tels que définis ci-dessus,
   - ou bien R₁₀ forme, avec l'atome d'azote auquel il est rattaché et un atome de carbone situé en une position quelconque de la structure cyclique de la formule (a), mais non adjacent audit atome d'azote, un pont comprenant de 3 à 5 chaînons,
   **R** et **R'** représentent indépendamment l'un de l'autre un atome d'hydrogène ou un groupe alkyle, cycloalkyle, hétérocycloalkyle, aryle, hétéroaryle, alkylaryle ou alkylhétéroaryle, ou bien R et R' peuvent former ensemble un cycloalkyle ou un hétérocycloalkyle.
   les groupes alkyles, cycloalkyles, hétérocycloalkyles, aryles et hétéroaryles étant éventuellement substitués par un ou plusieurs groupes choisis parmi les groupes R, R', - OR, -NRR', -CO-NRR', -NR-CO-R', -NR-CO-NRR', -NO₂, -CN et -COOR, OCOR, COR, OCONRR', NRCOOR'
**(2)** un groupe de formule -A-R₁₈, -A-CH=N-R₁₉, -A-N(R₂₀)-A'-R₁₉, -A-CO-N(R₂₀)-A'-R₁₉, -A-CH(NH₂)-R₁₉ ou -A-N(R₂₀)-COO-A', dans lesquels A et A' représentent un groupe alkyle linéaire ou ramifié, R₁₈ représente un atome d'halogène ou un groupe -NH₂, hydroxyle ou phényle, R₁₉ représente un atome d'hydrogène ou un groupe hydroxyle, phényle, benzyle ou hétéroaryle, et R₂₀ représente un atome d'hydrogène ou un groupe benzyle,
**(3)** un groupe de formule (d) : 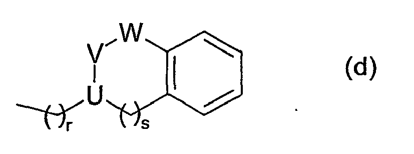 éventuellement substitué, en des positions quelconques, par 1 à 4 groupes R₇, identiques ou différents les uns des autres, tels que définis ci-dessus et dans laquelle r est égal à 1, 2 ou 3, s est égal à 0 ou 1, et l'un de U, V ou W représente un atome d'azote, les autres parmi U, V et W représentant des chaînons méthylène (c'est-à-dire des chaînons -CH₂- pour V et W et un chaînon -CH- pour U), ou
**(4)** un groupe -(CH₂)ᵣ-hétéroaryle, où r est égal à 1, 2 ou 3.

Les composés de formule (I) comportent au moins un atome de carbone asymétrique. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Parmi les composés de formule (I) objets de l'invention, on préfère ceux dans lesquels l'atome de carbone, identifié par l'astérisque * dans la formule suivante, présente une configuration (R) :

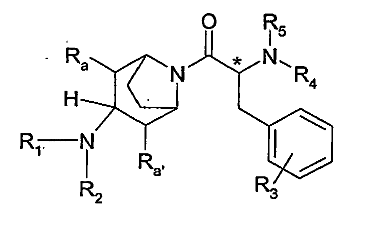

Les composés de formule (I) selon l'invention peuvent également exister sous forme de mélanges de conformères, qui font également partie de l'invention. Ils peuvent en outre exister sous forme d'isomères cis ou trans, ou sous forme d'isomères endo ou exo. Ces isomères ainsi que leur mélange, font partie de l'invention.

A cet égard, le noyau tropane des composés de formule (I) selon l'invention présente avantageusement une configuration endo, telle que représentée ci-dessous :

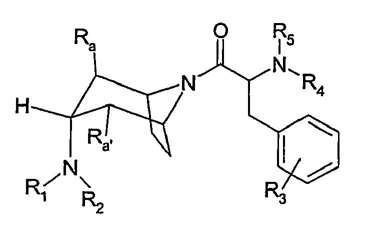

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, et sauf mention différente dans le texte, on entend par:
- un atome d'halogène : un fluor, un chlore, un brome ou un iode ;
   un groupe alkyle: un groupe aliphatique saturé ou insaturé (c'est-à-dire comprenant entre 1 et 3 insaturations de type éthylénique ou acétylénique), comprenant de 1 à 6 atomes de carbone, linéaire, cyclique ou ramifié. A titre d'exemples, on peut citer les groupes méthyle, éthyle, propyle, isopropyle, butyle, isobutyle, tertbutyle, pentyle, neo-pentyle, etc... et les groupes cycloalkyles définis ci-après, ainsi que les groupes alkyles cyclisés seulement en partie, tel que le groupe méthyl-cyclopropyle. Un tel groupe alkyle peut être substitué par 1 ou plusieurs groupes (par exemple par 1 à 6 groupes) choisis parmi les atomes d'halogène (résultant par exemple en un groupe -CF₃) et les groupes R, R', -OR, -NRR', -CO-NRR', -NR-CO-R', -NR-CO-NRR', -NO₂, -CN et - COOR, OCOR, COR, OCONRR', NRCOOR', où R et R' représentent indépendamment l'un de l'autre un atome d'hydrogène, un groupe alkyle, cycloalkyle, hétérocycloalkyle, aryle, hétéroaryle, alkylaryle ou alkylhétéroaryle, ou peuvent former ensemble un cycloalkyle ou hétérocycloalkyle ,
- un groupe cycloalkyle : un groupe alkyle cyclique comprenant entre 3 et 8 atomes de carbone, tous les atomes de carbone étant engagés dans la structure cyclique. A titre d'exemples, on peut citer les groupes cyclopropyle, cyclobutyle, cyclopentyle, cyclohexyle, etc ... Un tel groupe cycloalkyle peut être substitué comme décrit ci-dessus pour le groupe alkyle ;
- un groupe hétérocycloalkyle : un groupe cycloalkyle tel que défini ci-dessus, comprenant en outre entre 1 et 4 hétéroatomes, tels que l'azote, l'oxygène et/ou le soufre. Un tel groupe hétérocycloalkyle peut-être substitué comme décrit ci-dessus pour le groupe cycloalkyle et peut comprendre une ou plusieurs, par exemple 1 ou 2, insaturations éthyléniques ou acétyléniques. A titre d'exemples de groupes hétérocycloalkyles, on peut citer les groupes pipéridinyle et tétrahydropyrane ;
- un groupe alcoxy : un radical -O-alkyle, où le groupe alkyle est tel que précédemment défini ;
- un groupe aryle : un groupe aromatique cyclique comprenant entre 5 et 10 chaînons, par exemple un groupe phényle. Un tel groupe aryle peut être substitué par 1 ou plusieurs groupes (par exemple par 1 à 6 groupes) choisis parmi les atomes d'halogène (résultant par exemple en un groupe -CF₃) et les groupes alkyles, R, R', -OR, -NRR', -CO-NRR', -NR-CO-R', -NR-CO-NRR', -NO₂, -CN, COR et -COOR, où R et R' représentent indépendamment l'un de l'autre un atome d'hydrogène, un groupe alkyle, cycloalkyle, hétérocycloalkyle, aryle, hétéroaryle, alkylaryle ou alkylhétéroaryle ou peuvent former ensemble un cycloalkyle ou hétérocycloalkyle;
- un groupe alkylaryle : un groupe alkyle tel que défini ci-dessus, lui-même substitué par un groupe aryle tel que défini ci-dessus. Un tel groupe alkylaryle est par exemple un groupe benzyle ;
- un groupe hétéroaryle : un groupe aromatique cyclique comprenant entre 5 et 10 chaînons et comprenant entre 1 et 6 hétéroatomes, tels que l'azote, l'oxygène et/ou le soufre. A titre d'exemple on peut citer le groupe pyridinyle et le groupe fyryle. Un tel groupe hétéroaryle peut-être substitué comme décrit ci-dessus pour le groupe aryle
- un groupe alkylhétéroaryle : un groupe alkyle tel que défini ci-dessus, lui-même substitué par un groupe hétéroaryle tel que défini ci-dessus.

Parmi les composés de formule (I) objets de l'invention, on peut citer ceux dans lesquels Rₐ, R_{a'}, R₂, R₃, R₄ et R₅ sont tels que définis ci-dessus et R₁ représente un groupe alkyle, cycloalkyle, hétérocycloalkyle ou phényle. De façon avantageuse, R₁ représente un groupe cycloalkyle, tel qu'un groupe cyclohexyle.

Parmi les composés de formule (I) objets de l'invention, on peut également citer ceux dans lesquels Rₐ, R_{a'}, R₁, R₃, R₄ et R₅ sont tels que définis ci-dessus et R₂ est choisi parmi les groupes suivants : -CO-R₁₅, -CO-NR₁₆R₁₇, -CO-NR₁₅-NR₁₆R₁₇, -CO-aryle, -CO-hétéroaryle, -CO-(CH₂)_{x'}-NR₁₆R₁₇, -(CH₂)ₓ-NR₁₆R₁₇, -(CH₂)ₓ-OH, -(CH₂)ₓ-aryle, -(CH₂)ₓ-hétéroaryle, -(CH₂)_{x'}-CO-R₁₅ et -(CH₂)_{x'}-CO-NR₁₆R₁₇, dans lesquels :
- x = 0, 1, 2, 3 ou 4 et x' = 1, 2, 3 ou 4,
- R₁₅ représente un atome d'hydrogène ou un groupe alkyle, cycloalkyle ou alcoxy, et
- R₁₆ et R₁₇, identiques ou différents l'un de l'autre, représentent un atome d'hydrogène ou un groupe alkyle, cycloalkyle ou alcoxy, ou bien R₁₆ et R₁₇ forment ensemble, avec l'atome d'azote auquel ils sont rattachés, une structure mono- ou bicyclique comportant de 4 à 10 chaînons et comprenant éventuellement 1 à 3 hétéroatomes supplémentaires et/ou 1 à 3 insaturations éthyléniques ou acétyléniques, ce cycle étant éventuellement substitué en des positions quelconques par 1 à 3 groupes choisis parmi les atomes d'halogène et les groupes hydroxyles, alkyles, cycloalkyles et alcoxy.

Parmi les composés de formule (I) objets de l'invention, on peut plus particulièrement citer ceux dans lesquels R₂ représente un groupe -CO-NR₁₆R₁₇, où R₁₆ et R₁₇ représentent des groupes alkyles ou alcoxy. R₂ représente avantageusement un groupe -CO-NR₁₆R₁₇, ou R₁₆ et R₁₇ représentent des groupes alkyles.

Parmi les composés de formule (I) objets de l'invention, on peut également citer ceux dans lesquels Rₐ, R_{a'}, R₁, R₂, R₄ et R₅ sont tels que définis ci-dessus et R₃ représente 1 à 3 groupes, identiques ou différents les uns des autres, choisis parmi les atomes d'halogène. Avantageusement, R₃ représente un seul groupe, de préférence un atome de chlore.

Parmi les composés de formule (I) objets de l'invention, on peut également citer ceux dans lesquels Rₐ, R_{a'}, R₁, R₂, R₃ et R₅ sont tels que définis ci-dessus et R₄ est choisi parmi:
(1) un groupe de formule (a-1), (a-2), (a-3), (a-4) ou (b-1) ci-dessous : 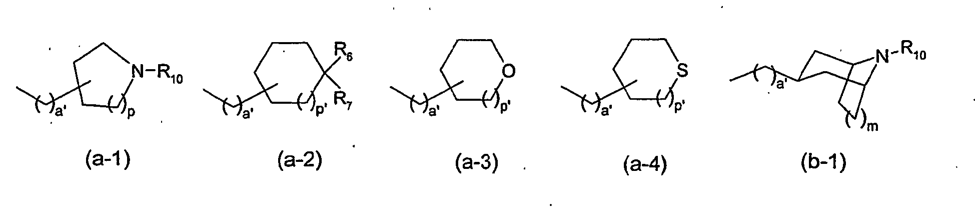 dans lesquelles chacun des cycles de formules (a-1), (a-2), (a-3), (a-4) et (b-1) peut être substitué, en des positions quelconques, par 1 à 4 groupes R₇, identiques ou différents les uns des autres, tels que définis ci-avant, et dans lesquelles a' = 0 ou 1, p = 0, 1, 2 ou 3; p' = 0 ou 1, m = 0, 1 ou 2 et R₆ et R₁₀ sont tels que définis ci-avant,
(2) un groupe de formule -A-R₁₈ ou -A-CH=N-R₁₉, où A, R₁₈ et R₁₉ sont tels que définis ci-avant,
(3) un groupe de formule (d) telle que définie ci-avant : 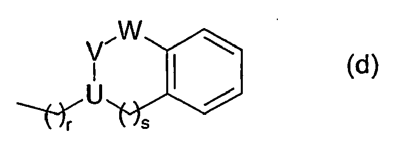
(4) un groupe -(CH₂)ᵣ-furyle ou -(CH₂)ᵣ-pyridinyle, où r est égal à 1, 2 ou 3.

Dans les groupes de formules (a-1), (a-2), (a-3), (a-4) ou (b-1) ci-dessus, on peut en particulier citer ceux pour lesquels R₆ représente un groupement -NH₂ ou phényle, R₇ représente un atome d'hydrogène ou un groupe hydroxyle et R₁₀ représente un atome d'hydrogène ou un groupe aryle, alkylaryle ou forme, avec l'atome d'azote auquel il est rattaché et un atome de carbone situé en une position quelconque de la structure cyclique de la formule (a-1), mais non adjacent audit atome d'azote, un pont comprenant de 3 à 5 chaînons. Avantageusement, R₁₀ représente un atome d'hydrogène dans le groupe de formule (b-1) et, dans le groupe de formule (a-1), un atome d'hydrogène ou un groupe phényle, benzyle ou forme, avec l'atome d'azote auquel il est rattaché et un atome de carbone situé en une position quelconque de la structure cyclique de la formule (a-1), mais non adjacent audit atome d'azote, un pont comprenant 4 chaînons.

Parmi les composés de formule (I) objets de l'invention, on peut encore citer ceux dans lesquels. Rₐ, R_{a'}, R₁, R₂, R₃ et R₅ sont tels que définis ci-dessus et R₄ est choisi parmi :
(1) un groupe de formule (a-5), (a-6) ou (b-2) ci-dessous : 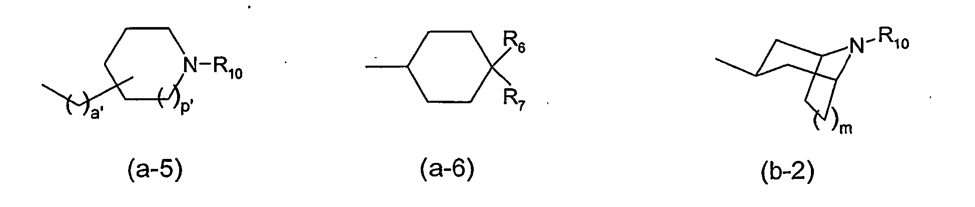 dans lesquelles chacun des cycles de formules (a-5), (a-6) et (b-2) peut être substitué, en des positions quelconques, par 1 à 4 groupes R₇, identiques ou différents les uns des autres, tels que définis ci-avant, et dans lesquelles a' = 0 ou 1, p'. = 0 ou 1, m = 0, 1 ou 2 et R₆ et R₁₀ sont tels que définis ci-avant,
(2) un groupe de formule -A-R₁₈ ou -A-CH=N-R₁₉, où A, R₁₈ et R₁₉ sont tels que définis ci-avant,
(3) un groupe de formule (d-1), où r = 1, 2 ou 3 : 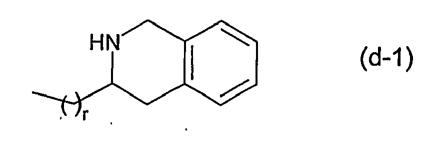
(4) un groupe -(CH₂)ᵣ-furyle ou -(CH₂)ᵣ-pyridinyle, où r est égal à 1, 2 ou 3.

Dans les groupes de formules (a-5), (a-6) et (b-2) ci-dessus, on peut en particulier citer ceux pour lesquels R₆ représente un groupement -NH₂ ou phényle, R₇ représente un atome d'hydrogène ou un groupe hydroxyle et R₁₀ représente un atome d'hydrogène ou un groupe aryle, alkylaryle ou forme, avec l'atome d'azote auquel il est rattaché et un atome de carbone situé en une position quelconque de la structure cyclique de la formule (a-5), mais non adjacent audit atome d'azote, un pont comprenant de 3 à 5 chaînons. Avantageusement, R₁₀ représente un atome d'hydrogène dans le groupe de formule (b-2) et, dans le groupe de formule (a-5), un atome d'hydrogène ou un groupe phényle ou benzyle ou forme, avec l'atome d'azote auquel il est rattaché et un atome de carbone situé en une position quelconque de la structure cyclique de formule (a-5), mais non adjacent audit atome d'azote, un pont comprenant 4 chaînons.

Parmi les composés de formule (I) objets de l'invention, on peut également citer ceux dans lesquels Rₐ, R_{a'}, R₁, R₂, R₃ et R₄ sont tels que définis ci-dessus et R₅ représente un atome d'hydrogène ou un groupe alkyle comprenant de 1 à 4 atomes de carbone. R₅ représente de préférence un atome d'hydrogène.

Parmi les composés de formule (I) objets de l'invention, on peut également citer ceux dans lesquels R₁, R₂, R₃, R₄ et R₅ sont tels que définis ci-dessus et Rₐ et R_{a'} représentent des atomes d'hydrogène ou des groupes alkyles comprenant de 1 à 4 atomes de carbone. Avantageusement, Rₐ et R_{a'} représentent indépendamment l'un de l'autre des atomes d'hydrogène ou des groupes méthyles. De façon préférée, Rₐ = R_{a'} = H.

Parmi les groupes R₆ définis précédemment, on peut notamment citer ceux dans lesquels R₆ représente un atome d'hydrogène ou un groupe -NR₈R₉ ou aryle, dans lesquels R₈ et R₉ représentent un atome d'hydrogène ou un groupe alkyle. On peut également citer les groupes R₆ représentant un atome d'hydrogène ou un groupe -NH₂ ou phényle.

Parmi les groupes R₇ définis précédemment, on peut notamment citer ceux dans lesquels R₇ représente un atome d'hydrogène ou d'halogène ou un groupe alkyle, hydroxyle (correspondant à un groupe -OR, où R représente un atome d'hydrogène) ou alcoxy (correspondant à un groupe -OR, où R représente un groupe alkyle). R₇ représente avantageusement un atome d'hydrogène ou un groupe hydroxyle.

Parmi les groupes R₈ et R₉ définis précédemment, on peut notamment citer ceux dans lesquels R₈ et R₉ représentent un atome d'hydrogène ou un groupe alkyle.

Parmi les groupes R₁₀ définis précédemment, on peut notamment citer ceux dans lesquels R₁₀ représente un atome d'hydrogène ou un groupe aryle (tel qu'un groupe phényle) ou alkylaryle (tel qu'un groupe benzyle), ou bien R₁₀ forme, avec l'atome d'azote qui le porte et un atome de carbone situé en une position quelconque de la structure cyclique à laquelle il est rattaché, mais non adjacent audit atome d'azote, un pont comprenant de 3 à 5 chaînons.

Parmi les groupes R et R' définis précédemment, on peut notamment citer ceux dans lesquels R et R' représentent un atome d'hydrogène ou un groupe alkyle.

Chacune des définitions données ci-avant pour les groupes Rₐ, R_{a'}, R_{1,} R₂, R₃, R₄, R₅, R₆, R₇, F₈, R₉, R₁₀, R et R' peuvent se combiner les unes avec les autres de façon à obtenir différents sous-groupes de composés de formule (I) selon la présente invention.

On peut par exemple citer un sous-groupe de composés de formule (I) selon l'invention, dans lequel :
- Rₐ et R_{a'} représentent indépendamment l'un de l'autre des atomes d'hydrogène
ou des groupes méthyles,
- R₁ représente un groupe alkyle, cycloalkyle ou hétérocycloalkyle,
- R₂ est choisi parmi les groupes suivants : -CO-R₁₅, -CO-NR₁₆R₁₇, -CO-NR₁₅-NR₁₆R₁₇, -CO-aryle, -CO-hétéroaryle, -CO-(CH₂)ₓ-NR₁₆R₁₇, -(CH₂)ₓ-NR₁₆R₁₇, -(CH₂)ₓ-OH, -(CH₂)ₓ-aryle, -(CH₂)ₓ-hétéroaryle, -(CH₂)_{x'}-CO-R₁₅ et -(CH₂)_{x'}-CO-NR₁₆R₁₇, dans lesquels :
   - x = 0, 1, 2, 3 ou 4 et x' = 1, 2, 3 ou 4,
   - R₁₅ représente un atome d'hydrogène ou un groupe alkyle, cycloalkyle ou alcoxy, et
   - R₁₆ et R₁₇, identiques ou différents l'un de l'autre, représentent un atome d'hydrogène ou un groupe alkyle, cycloalkyle ou alcoxy, ou bien R₁₆ et R₁₇ forment ensemble, avec l'atome d'azote auquel ils sont rattachés, une structure mono- ou bicyclique comportant de 4 à 10 chaînons et comprenant éventuellement 1 à 3 hétéroatomes supplémentaires et/ou 1 à 3 insaturations éthyléniques ou acétyléniques, ce cycle étant éventuellement substitué, en des positions quelconques par 1 à 3 groupes choisis parmi les atomes d'halogène et les groupes hydroxyles, alkyles, cycloalkyles et alcoxy,
- R₃ représente 1 à 3 groupes, identiques ou différents les uns des autres, choisis parmi les atomes d'halogène,
- R₄ est choisi parmi :
   (1) un groupe de formule (a-1), (a-2), (a-3), (a-4) ou (b-1) ci-dessous : 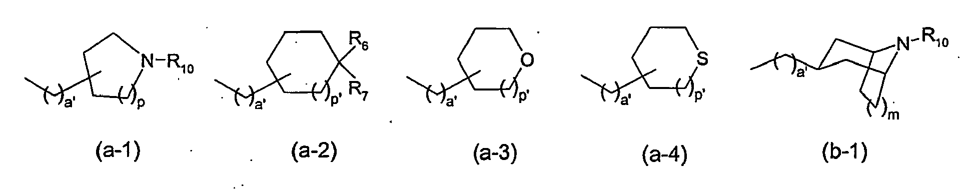 dans lesquelles chacun des cycles de formules (a-1), (a-2), (a-3), (a-4) et (b-1) peut être substitué; en des positions quelconques, par 1 à 4 groupes R₇; identiques ou différents les uns des autres, tels que définis ci-avant, et dans lesquelles a' = 0 ou 1, p = 0, 1, 2 ou 3, p' = 0 ou 1, m = 0, 1 ou 2 et R₆ et R₁₀ sont tels que définis ci-avant,
   (2) un groupe de formule -A-R₁₈ ou -A-CH=N-R₁₉, où A, R₁₈ et R₁₉ sont tels que définis ci-avant,
   (3) un groupe de formule (d) telle que définie ci-avant : 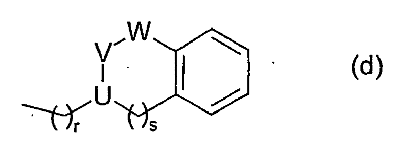
   (4) un groupe -(CH₂)ᵣ-furyle ou -(CH₂)ᵣ-pyridinyle, où r est égal à 1, 2 ou 3, et
   - R₅ représente un atome d'hydrogène.

Selon un autre objet l'invention se rapporte aux composés préférés en particulier les composés de configuration endo, dont les noms suivent :
Dans les listes qui suivent les chiffres devant les nomenclatures des produits correpondent aux n° d'exemple des composés du tableau
   1 : *N*-[8-(4-chloro-*N*-piperidin-4-yl-D-phenylalanyl)-8-azabicyclo[3.2.1]oct-3-yl]-*N-*cyclohexyl-*N*',*N*'-diethylurea
   2: *N*-[8-(4-chloro-*N*-piperidin-3-yl-D-phenylalanyl)-8-azabicyclo[3.2.1]oct-3-yl]-*N* cyclohexyl-*N*',*N*'-diethylurea
   3: *N*-{8-[*N*-(4-aminocyclohexyl)-4-chloro-D-phenylalanyl]-8-azabicyclo[3.2.1]oct-3-yl}-*N*-cyclohexyl-*N*',*N*'-diethylurea
   4: *N*-{8-[4-chloro-*N*-(tetrahydro-2*H*-pyran-4-yl)-D-phenylalanyl]-8-azabicyclo[3.2.1]oct-3-yl}-*N*-cyclohexyl-*N*',*N*'-diethylurea
   5: *N*-[8-(*N*-8-azabicyclo[3.2.1]oct-3-yl-4-chloro-D-phenylalanyl)-8-azabicyclo[3.2.1]oct-3-yl]-*N*-cyclohexyl-*N*',*N*'-diethylurea
   6: *N*-{8-[4-chloro-*N*-(piperidin-4-ylmethyl)-D-phenylalanyl]-8-azabicyclo[3.2.1]oct-3-yl}-*N*-cyclohexyl-*N*,*N*'-diethylurea
   7: *N*-{8-[4-chloro-*N*-(piperidin-2-ylmethyl)-D-phenylalanyl]-8-azabicyclo[3.2.1]oct-3-yl)-*N*-cyclohexyl-*N*',*N*'-diethylurea
   8: *N*-{8-[4-chloro-*N*-(tetrahydro-3-thienyl)-D-phenylalanyl]-8-azabicyclo[3.2.1]oct-3-yl}-*N*-cyclohexyl-*N'*,*N'*-diethylurea
   9: *N*-[8-(*N*-1-azabicyclo[2.2.2]oct-3-yl-4-chloro-D-phenylalanyl)-8-azabicyclo[3.2.1]oct-3-yl]-*N*-cyclohexyl-*N*',*N*'-diethylurea
   10: *N*-[8-(*N*-azepan-4-yl-4-chloro-D-phenylalanyl)-8-azabicyclo[3.2.1]oct-3-yl]-*N-*cyclohexyl-*N*',*N*'-diethylurea
   11: *N*-[8-(4,chloro-*N*-{[(2*S*,4*R*)-4-hydroxypyrrolidin-2-yl]methyl}-D-phenylalanyl)-8-azabicyclo[3.2.1]oct-3-yl]-*N*-cyclohexyl-*N*',*N'*-diethylurea
   12: *N*-[8-(4-chloro-*N*-{[(2*R*,4*R*)-4-hydroxypyrrolidin-2-yl]methyl}-D-phenylalanyl)-8- - azabicyclo[3.2.1]oct-3-yl]-*N*-cyclohexyl-*N'*,*N'*-diethylurea
   13: *N*-[8-(4-chloro-*N*-{((2*R*,4*S*)-4-hydroxypyrrolidin-2-yl]methyl}-D-phenylalanyl)-8-azabicyclo[3.2.1]oct-3-yl]-*N*-cyclohexyl-*N'*,*N'*-diethylurea
   14 : *N*-{8-(4-chloro-*N*-(1-phenylpiperidin-4-yl)-D-phenylalanyl]-8-azabicyclo[3.2.1]oct-3-yl}-*N*-cyclohexyl-*N*,*N*-diethylurea
   15: *N*-(8-{*N*-[(1-benzylpyrrolidin-3-yl)methyl]-4-chloro-D-phenylalanyl)-8-azabicyclo[3.2.1]oct-3-yl)-*N*-cyclohexyl-*N*,*N*-diethylurea
   16: *N*-[8-(4-chloro-*N*-pyrrolidin-3-yl-D-phenylalanyl)-8-azabicyclo[3.2.1]oct-3-yl]-*N* cyclohexyl-*N*',*N'*-diethylurea
   17: *N*-(8-{4-chloro-*N*-[4-(4-hydroxyphenyl)cyclohexyl]-D-phenylanyl}-8-azabicyclo[3.2.1]oct-3-yl)-*N*-cyclohexyl-*N*,*N*-diethylurea
   18: *N*-(8-[*N*-(2-aminoethyl)-4-chloro-D-phenylalanyl]-8-azabicyclo[3.2.1]oct-3-yl}-*N*-cyclohexyl-*N*',*N*'-diethylurea
   19: *N*-{8-[*N*-(3-aminopropyl)-4-chloro-D-phenylalanyl]-8-azabicyclo[3.2.1]oct-3-yl}-*N*-cyclohexyl-*N*',*N*'-diethylurea
   20: *N*-(8-{4-chloro-*N*-[(2E)-2-(hydroxyimino)-1-methylethyl]-D-phenylalanyl}-8-azabicyclo[3.2.1]oct-3-yl)-*N*-cyclohexyl-*N*',*N*'-diethylurea
   21: *N*-{8-[4-chloro-*N*-(2-fluoro-1-methylethyl)-D-phenylalanyl]-8-azabicyclo[3.2.1]oct-3-yl}-*N*-cyclohexyl-*N*',*N*'-diethylurea
   22: *N*-(8-{4-chloro-*N*-[(3R)-1,2,3,4-tetrahydroisoquinolin-3-ylmethyl]-D-phenylalanyl}-8-azabicyclo[3.2.1]oct-3-yl)-*N*-cyclohexyl-*N*',*N*'-diethylurea
   23: *N-*{8-[4-chloro-*N*-(pyridin-2-ylmethyl)-D-phenylalanyl]-8-azabicyclo[3.2.1]oct-3-yl}-*N*-cyclohexyl-*N*',*N*'-diethylurea
   24: *N*-{8-[4-chloro-*N*-(2-furylmethyl)-D-phenylalanyl]-8-azabicyclo[3.2.1]oct-3-yl}-*N*-cyclohexyl-*N*',*N*'-diethylurea
   25: *N*-(8-{4-chloro-*N*-[(2*R*)-pyrrolidin-2-ylmethyl]-D-phenylalanyl}-8-azabicyclo[3.2.1]oct-3-yl)-*N*-cyclohexyl-*N*',*N*'-diethylurea
   26: *N*-(8-{4-chloro-*N*-[(2*S*)-pyrrolidin-2-ylmethyl]-D-phenylalanyl}-8-azabicyclo[3.2.1]oct-3-yl)-*N*-cyclohexyl-*N*',*N*'-diethylurea
   27: *N*-[8-(*N*-azetidin-3-yl-4-chloro-D-phenylalanyl)-8-azabicyclo[3.2.1]oct-3-yl]-*N-*cyclohexyl-*N*',*N*'-diethylurea
   28: *N*-(8-{*N*-[(1-benzylpyrrolidin-3-yl)methyl]-4-chloro-D-phenylalanyl}-8-azabicyclo[3.2.1]oct-3-yl)-*N*-cyclohexyl-*N*',*N*'-diethylurea

Dans ce qui suit, on entend par groupe protecteur (Pg) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green W. et al., 1999, 3rd Edition (John Wiley & Sons, Inc., New York).

On entend par groupe partant (Lg), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « March's Advanced Organic Chemistry », J. March et al., 5th Edition, 2001, EMInter Ed.

On entend par groupement Boc un groupement t-butoxycarbonyl, Bn un groupement benzyle, CBz un groupement benzyloxycarbonyl, Fmoc un groupement 9-fluorenylmethyl-carbamate, et par h les heures

Conformément à l'invention, on peut préparer les composés de formule générale (I) selon le procédé présenté dans le schéma 1.

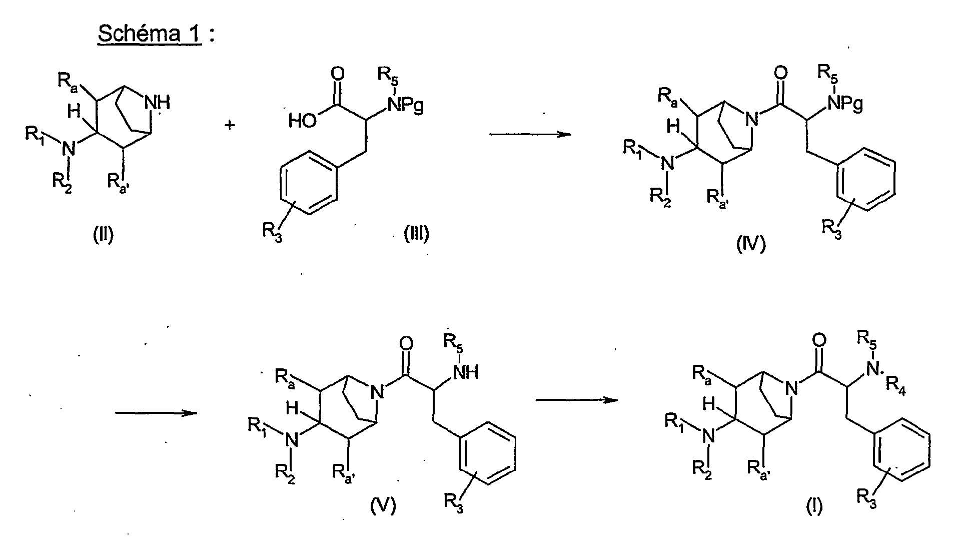

Selon le schéma 1, les composés de formule (IV) peuvent être préparés par couplage entre les intermédiaires de formule (II) et un aminoacide de formule (III) dont la fonction amine est protégée par un groupement protecteur Pg (par exemple un groupement Boc, CBz ou Fmoc), dans des conditions de couplage peptidique classiques, en utilisant par exemple comme agent couplant le dicyclocarbodiimide, le chlorhydrate du 1-(3-diméthylaminopropyl)-3-éthylcarbodiimide ou le bromo-tris-pyrrolidino-phosphonium hexafluorophosphate, en présence ou non d'hydroxybenzo-triazole, et comme base la triéthylamine ou la diisopropyléthylamine dans un solvant tel que le dioxane, le dichlorométhane ou l'acétonitrile.

Les aminoacides de formule générale (III) sont disponibles commercialement ou peuvent être préparés par des méthodes décrites dans la littérature (Williams, R.M., Synthesis of Optically Active α-Aminoacids, Pergamon Press, Oxford, 1989).

Les composés de formule (V) sont obtenus par déprotection de la fonction amine des composés de formule (IV), par des méthodes choisies parmi celles connues de l'Homme de l'art. Elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, l'hydrogénation avec le métal approprié dans le méthanol ou l'éthanol dans le cas d'un CBz, et de pipéridine pour un groupement Fmoc, à des températures variant de -10°C à 100°C.

Dans une dernière étape, les composés de formule (I) sont obtenus par amination réductrice, réalisée en mettant les composés de formule (V) en présence d'un dérivé du groupe R₄ de type oxo (aldéhyde ou cétone), en utilisant un réducteur tel que le borohydrure de sodium, le triacétoxyborohydrure de sodium ou le cyanoborohydrure de sodium, en présence ou non d'un acide de Bronsted (tel que l'acide chlorhydrique) ou de Lewis (tel que le tétraisopropoxyde de titane) dans un solvant tel que le dichloroéthane, le dichlorométhane, l'acide acétique ou le méthanol, à des températures comprises entre -10°C et 30°C. Les aldéhydes dérivés du groupe R₄, quand ils ne sont pas commerciaux, sont préparés à partir des acides correspondants par des méthodes connues de l'Homme de l'art, par exemple par transformation en amide de Weinreb dans des conditions de couplage peptidique, puis par réduction de celui-ci par un hydrure tel que l'hydrure de lithium aluminium.

Les dérivés du groupe R₄ de type cétone ou aldéhyde peuvent être commerciaux ou obtenus par des méthodes connues de l'Homme de l'art, par exemple par acylation de la fonction amine ou hydroxyle libre du dérivé de type cétone.

Les composés de formule générale (I), dans lesquels R₄ répond aux formules (a), (b), (c), (d), -A-R₁₈, -A-N(R₂₀)-A'-R₁₉ et -A-CH(NH₂)-R₁₉, peuvent également être préparés selon le procédé présenté dans le schéma 2.

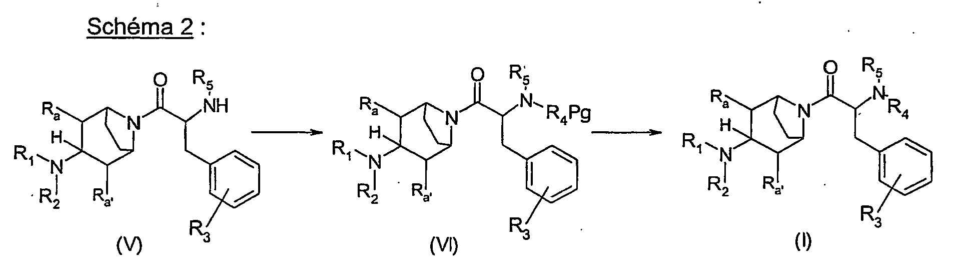

Selon le schéma 2, les composés de formule (V), obtenus comme décrit précédemment dans le schéma 1, sont mis en présence avec un dérivé du groupe R₄ de type oxo (réaction d'amination réductrice, comme décrit ci-dessus en rapport avec le schéma 1), ledit groupe R₄ portant un groupe Pg protecteur d'amine, pour donner les composés dé formule (VI). La fonction amine des composés de formule (VI) est ensuite déprotégée par des méthodes connues de l'Homme de l'art, comme décrit précédemment.

Alternativement, les composés de formule (VI) conduisant aux composés de formule (I) peuvent être préparés selon le procédé présenté dans le schéma 3

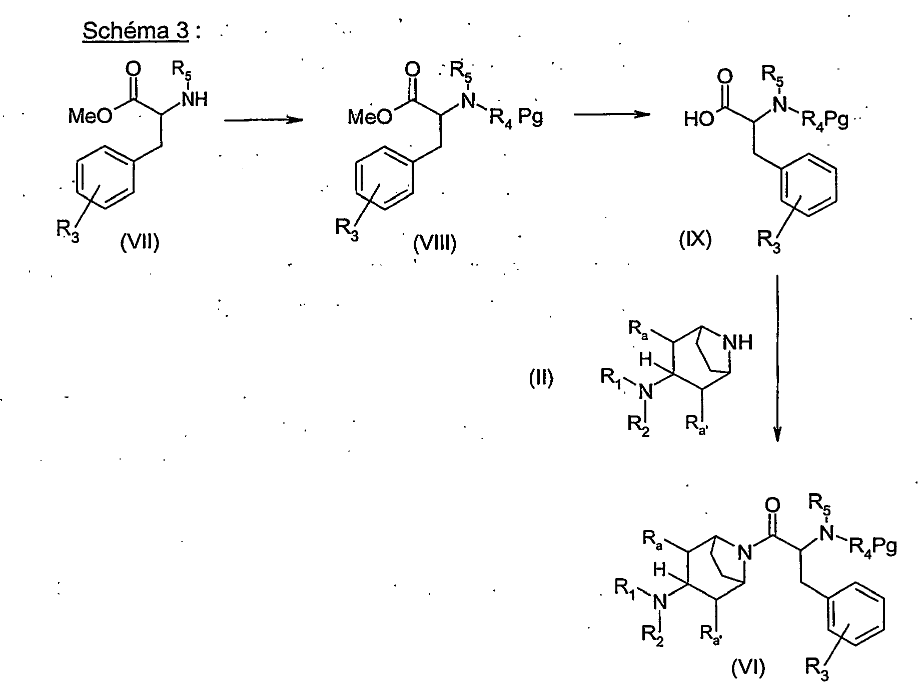

Selon le schéma 3, les composés de formule (VIII) peuvent être obtenus par amination réductrice, comme décrit précédemment, réalisée à partir des aminoacides de formule (VII). L'aminoacide de formule (VII) est disponible commercialement quand R₅ = H, ou peut être préparé par des méthodes décrites dans la littérature (Williams, R.M., Synthesis of Optically Active α-Aminoacids, Pergamon Press, Oxford, 1989). Dans les cas où R₅ représente un groupe alkyle, les aminoacides de formule (VII) peuvent être préparés par alkylation de l'aminoacide commercial protégé sur la fonction amine, selon les méthodes d'alkylation connues de l'Homme de l'art.

Les composés de formule (IX) peuvent être synthétisés par saponification des esters de formule (VIII), par exemple en présence d'hydroxyde de sodium ou d'hydroxyde de lithium dans un solvant tel que le méthanol, le tétrahydrofurane ou l'eau, ou un mélange de ces solvants.

Les composés de formule générale (VI) peuvent être préparés par couplage peptidique entre les intermédiaires de formule (II) et l'aminoacide de formule (IX), dans des conditions de couplage peptidique telles que décrites dans le schéma 1.

Les composés de formulés (II) peuvent être obtenus selon le procédé présenté dans le schéma 4.

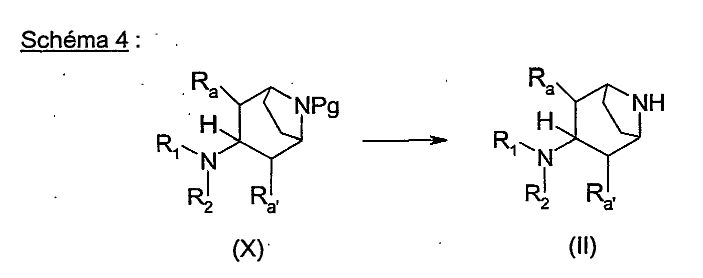

Selon le schéma 4, les composés de formule (II) peuvent être préparés à partir du composé de formule (X) (où Pg est un groupement protecteur d'amine, tel que défini dans le schéma 1), après déprotection de la fonction amine par des méthodes choisies parmi celles connues de l'Homme de l'art, comme décrit précédemment.

Le composé de formule (X) est préparé selon les méthodes décrites dans la littérature ou connues de l'Homme de l'art, adaptées en fonction de la nature des groupes R₁ et R₂. Les schémas 5 à 9 ci-après présentent des exemples de préparation des composés de formule (X) selon différentes natures du groupe R₂. Par exemple dans le cas où R₂ représente un groupe -CO-R₁₅, où R₁₅ est tel que défini ci-avant, la préparation du composé (Xa) correspondant peut être effectuée selon le schéma 5.

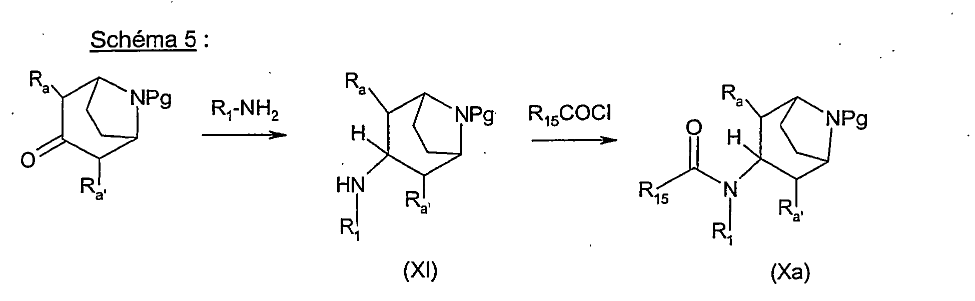

Selon le schéma 5, les composés de formule (XI) peuvent être obtenus par amination réductrice, dans les conditions décrites précédemment, de la nortropanone dont la fonction amine est protégée (par exemple la Boc-nortropanone commerciale). On obtient ensuite les composés de formule (Xa) par réaction des composés de formule (XI) avec un chlorure d'acide de formule R₁₅COCl, en présence d'une base organique telle que la triéthylamine ou la pyridine, dans un solvant tel que le dichlorométhane ou le tétrahydrofurane.

Le schéma 6 présente une voie de préparation des composés de formule (Xb) et (Xc), qui correspondent respectivement aux composés de formule (X) dans lesquels R₂ représente un groupe -CO-NR₁₆R₁₇ et -CO-NR₁₅-NR₁₆R₁₇, où R₁₅, R₁₆ et R₁₇ sont tels que définis ci-avant.

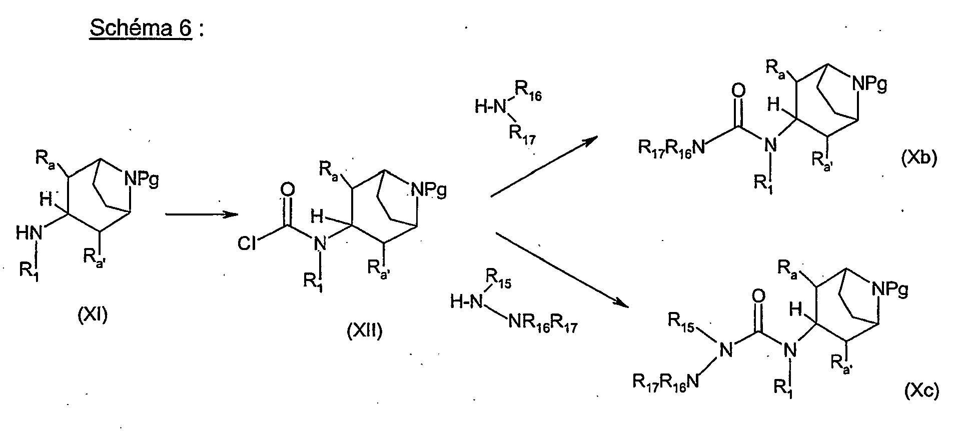

Selon le schéma 6, les composés de formule (XII) peuvent être préparés à partir des composés de formule (XI) par réaction avec du phosgène, du triphosgène ou du trichlorométhyle chloroformate dans le dichlorométhane ou le toluène en présence de triéthylamine ou de pyridine et une amine à des températures variant de -10°C et 80°C. La réaction des composés de formule (XII) avec une amine de formule HN(R₁₆)(R₁₇) ou une hydrazine de formule HN(R₁₅)(NR₁₆R₁₇) aboutit respectivement aux composés de formules (Xb) et (Xc).

Le schéma 7 présente une voie de préparation des composés de formule (Xd), correspondant aux composés de formule (X) dans lesquels R₂ représente un groupe -(CH₂)ₓ-NR₁₆R₁₇, où x = 2, 3 ou 4 et où R₁₆ et R₁₇ sont tels que définis ci-avant.

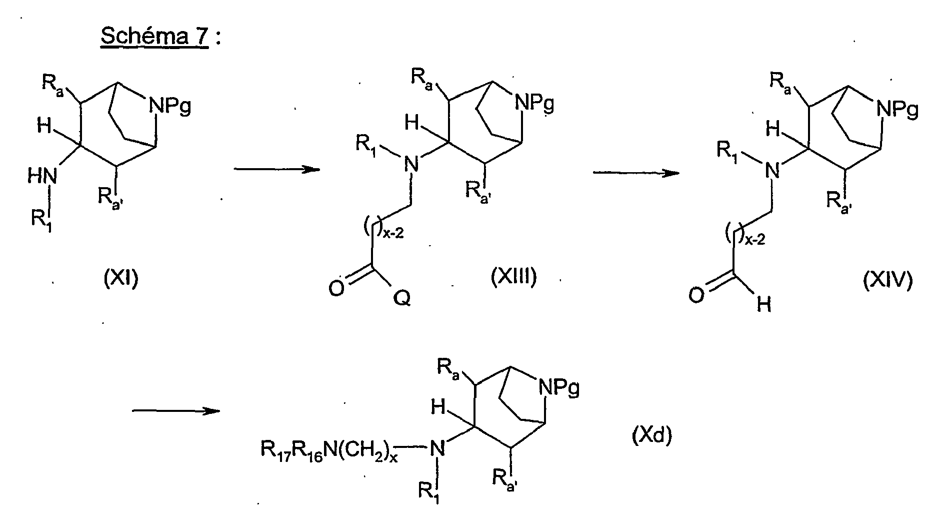

Selon le schéma 7, les composés de formule (XIII) peuvent être obtenus par amination réductrice réalisée sur les composés de formule (XI) en présence d'un aldéhyde de formule Q-CO-(CH₂)ₓ₋₂-CHO, où Q représente un groupe -O-alkyle ou -N(O-alkyle)(alkyle), en utilisant un réducteur tel que décrit précédemment en rapport avec le schéma 1.

Puis les composés de formule générale (XIII) peuvent être réduits pour conduire aux aldéhydes de formule (XIV), en utilisant un réducteur tel que l'hydrure de diisobutyl aluminium ou le tétraaluminohydrure de sodium dans le cas où Q est un groupe -O-alkyle, ou par réduction avec l'hydrure de lithium aluminium dans le cas où Q est un groupe -N(O-alkyle)(alkyle) (par exemple -N(OMe)Me), obtenu par exemple par réaction d'un organomagnésien, tel que le chlorure de diisopropylmagnésien, sur les composés de formule (XIII) où Q = -O-alkyle en présence d'une alkylhydroxylalkyle amine telle que la N,O-diméthylhydroxylamine, dans des solvants tels que le tétrahydrofurane ou le diéthyléther à des températures variant de -78°C à 20°C.

Les composés de formule (Xd) peuvent ensuite être préparés par amination réductrice réalisée en présence d'une amine de formule R₁₇R₁₆NH, en utilisant un réducteur tel que décrit ci-dessus.

Le schéma 8 présente une voie de préparation des composés de formule (Xe), correspondant aux composés de formule (X) dans lesquels R₂ représente un groupe -(CH₂₎ₓ-aryle (où x = 0, 1, 2, 3 ou 4) ou -(CH₂)ₓ-hétéroaryle (où x = 1, 2, 3 ou 4).

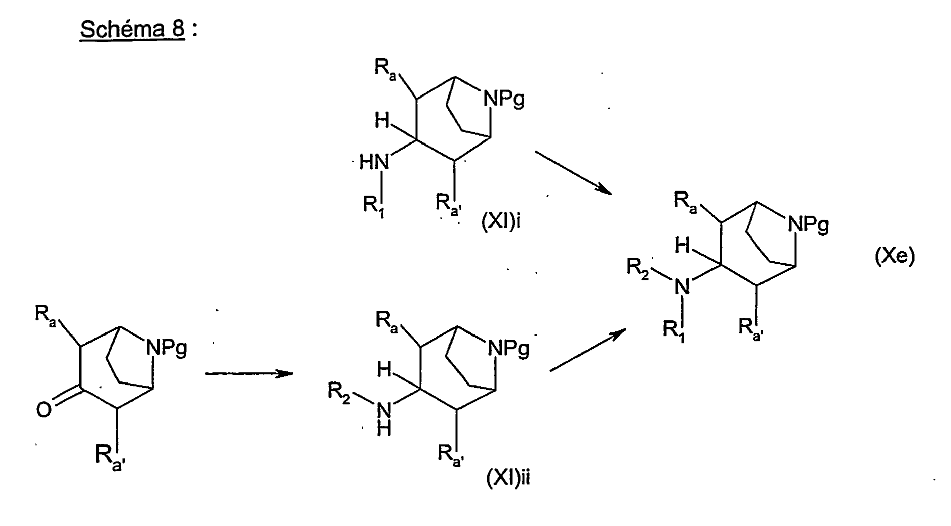

Selon le schéma 8, les composés de formules (Xe) dans lesquels R₂ représente un groupe -(CH₂)ₓ-hétéroaryle (où x = 1, 2 ou 3) peuvent être obtenus par amination réductrice à partir des composés de formule (XI)i, réalisée en présence d'un aldéhyde de formule : hétéroaryle-(CH₂)ₓ₋₁-CHO, en utilisant un réducteur tel que décrit précédemment en rapport avec le schéma 1.

La même réaction peut également être utilisée pour obtenir les composés de formule (Xd), et ce en utilisant un aldéhyde de formule R₁₇R₁₆N-(CH₂₎ₓ₋₁-CHO.

Les composés de formules (XI)ii dans lesquels R₂ représente un groupe -(CH2)ₓ-aryle (où x = 0, 1, 2 ou 3) peuvent être obtenus par amination réductrice à partir de la nortropanone protégée sur la fonction amine (telle que la Boc-nortropanone), réalisée en présence d'une amine de formule : aryle-(CH₂)ₓ-NH₂, en utilisant un réducteur tel que décrit précédemment en rapport avec le schéma 1. Les composés de formules (Xe) dans lesquels R₂ représente un groupe -(CH₂)ₓ-aryle peuvent ensuite être obtenus par amination réductrice à partir des composés de formule (XI)ii, réalisée en présence d'un dérivé de groupe R₁ de type oxo.

Le schéma 9 détaille une alternative pour la synthèse des composés de formule (Xe) dans lesquels R₂ représente un groupe -(CH₂)ₓ-hétéroaryle, où x est égal à 2 ou 3.

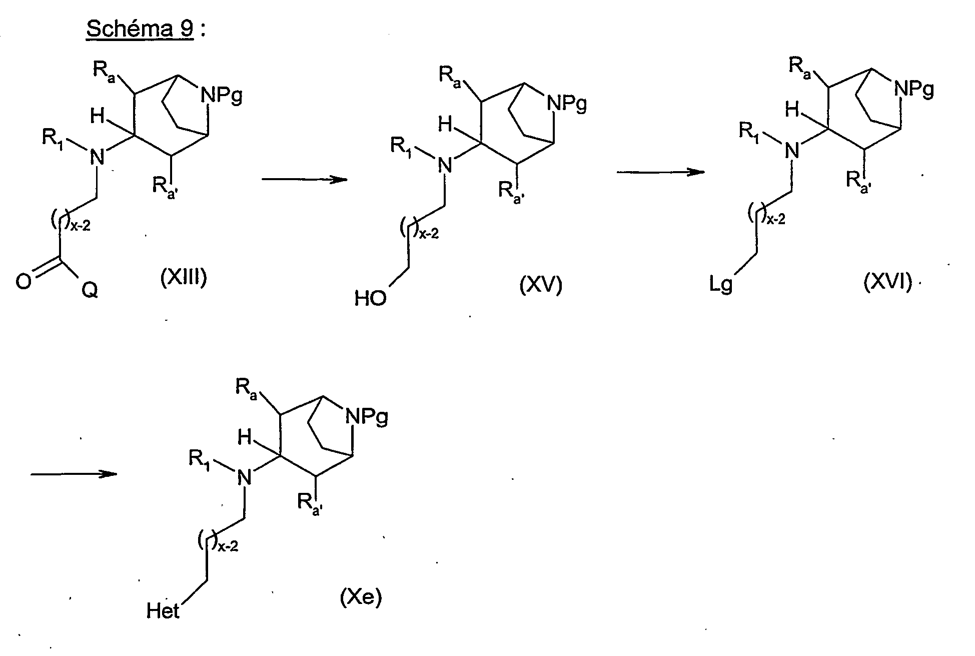

Selon le schéma 9, les composés de formule (XIII), dans lesquels Q représente un groupe -O-alkyle, peuvent être réduits en alcools correspondants en utilisant un réducteur tel que l'hydrure de lithium aluminium dans un solvant tel que le diéthyléther ou le tétrahydrofurane, à des températures variant de -60°C à 20°C.

Le groupement hydroxyle des composés de formule (XV) est ensuite transformé en groupement partant (Lg), tels que le chlorure ou le mésylate, par exemple par action de tétrabromométhane et de triphénylphosphine dans un solvant tel que le dichlorométhane ou par action de chlorure de méthanesulfonyle en présence d'une base organique telle que la triéthylamine à des températures variant de -20°C à la température ambiante, pour conduire aux composés de formule (XVI).

Les composés de formule (Xe) sont ensuite synthétisés par une réaction de substitution nucléophile entre les composés de formule (XVI) et l'anion d'un hétéroaryle (groupe « Het »).

Le schéma 10 présente une voie de préparation des composés de formule (I), dans lesquels R₄ représente un groupe de formule (d) tel que défini précédemment, où r = 1, 2 ou 3, s = 1 et U représente un atome d'azote.

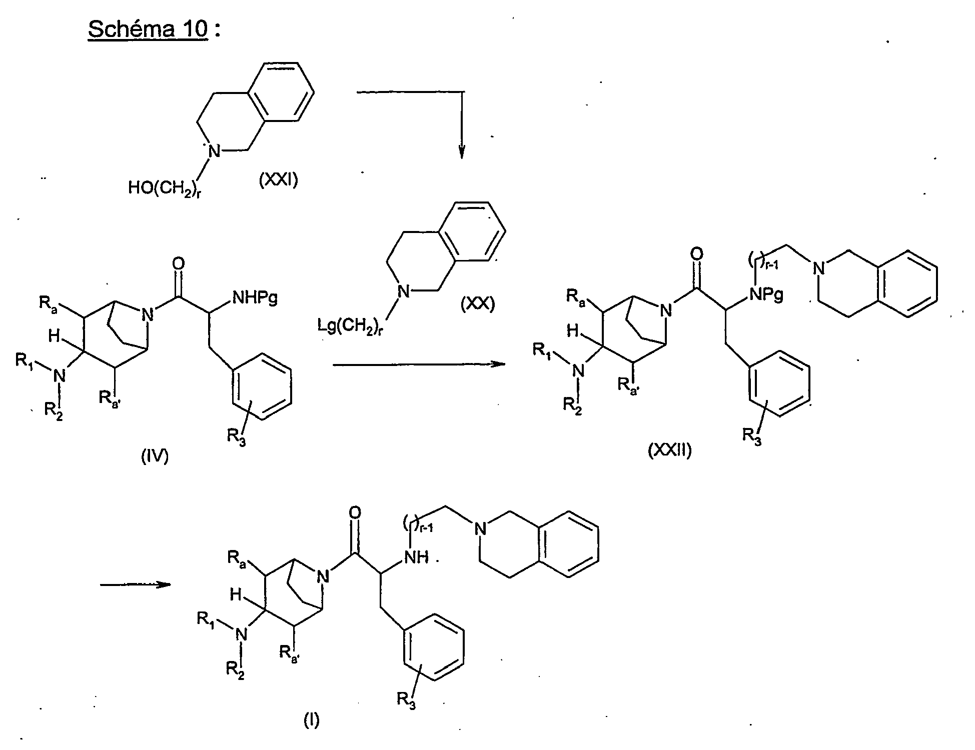

Les composés de formule (XXII) peuvent être obtenus par alkylation des composés de formule (IV) [où R₅ = H] par des tétrahydroisoquinolines de formule (XX), en substituant le groupe partant (Lg) des composés de formule (XX) par l'anion des composés de formule (IV), formé par réaction avec l'hydrure de sodium dans un solvant tel que le diméthylformamide à des températures variant de -20°C à 60°C, ou par réaction avec le diisopropylamidure de lithium dans un solvant tel que le tétrahydrofurane ou le diéthyléther à des températures variant de -78°C à 25°C.

Les composés de formule (XX) sont préparés dans des conditions classiques, comme par transformation du groupement hydroxyle des composés de formule (XXI) en groupe partant, tels que le chlorure ou le mésylate, par exemple par action de tétrabromométhane et de triphénylphosphine dans un solvant tel que le dichlorométhane ou par action de chlorure de méthanesulfonyle en présence d'une base organique telle que la triéthylamine à des températures variant de -20°C à la température ambiante. Les composés de formule (XXI) sont synthétisés selon les méthodes d'alkylation connues de l'Homme de l'art de la 1,2,3,4-tétrahydroisoquinoline commerciale.

La fonction amine des composés de formule (XII) est déprotégée, dans des conditions telles que décrites dans le schéma 1, pour aboutir aux composés de formule (I) [où R₅ = H et R₄ = 2-alkyl-1,2,3,4-tétrahydroisoquinoline].

Selon une variante du schéma 1, dans le cas où les composés de formule (I) comprennent, en tant que groupe R₄, un groupe de formule (a) de type cyclohexyle, c'est-à-dire un groupe de formule (a) où a = 0, p = 2 et X = -C(R₆)(R₇)-, où R₆ représente un groupe -OR₈, R₇ et R₈ étant tels que définis ci-avant, alors la préparation des composés de formule (I) peut être réalisée comme décrit dans le schéma 11.

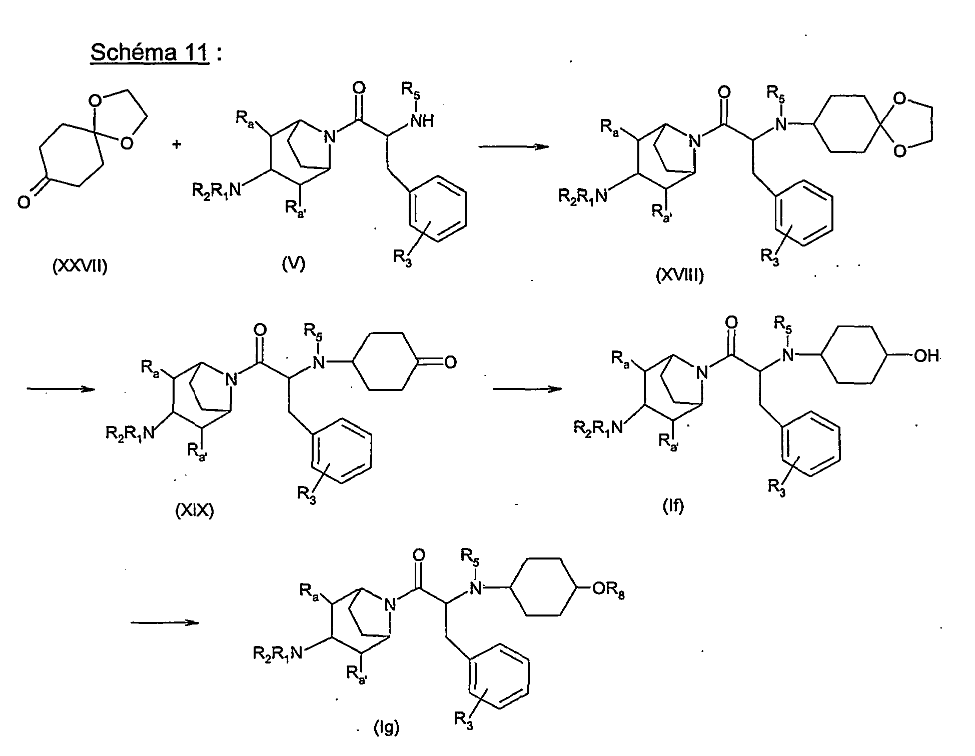

Selon le schéma 11, les composés de formule (XVIII) peuvent être obtenus par une amination réductrice entre le composé de formule (XVII) commercial et les composés de formule (V), dans des conditions telles que décrites dans le schéma 1.

La déprotection de la fonction oxo du composé de formule (XVIII) en présence d'un acide tel que l'acide chlorhydrique ou le pyridinium tosylate dans le tétrahydrofurane ou l'acétone à des températures comprises entre 0°C et 80°C conduit au composé de formule (XIX).

Les composés de formule (If) sont préparés par réduction des composés de formule (XIX) dans des conditions telles que décrites dans le schéma 6.

Quand R₈ est différent d'un atome d'hydrogène, on réalise une fonctionnalisation des composés de formule (If), par exemple une alkylation en présence d'une base telle que l'hydrure de sodium et d'un dérivé du groupe R₈ comportant un groupement partant Lg, ce qui aboutit aux composés de formule (lg).

Dans les schémas 1 à 11, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme de l'art.

L'invention, selon un autre de ses aspects, a également pour objet les composés de formules (II), (IV), (V), (Vl), (VIII), (IX), (X), (XVIII) et (XIX) : ces composés sont utiles comme intermédiaires de synthèse des composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1: N-[8-(4-chloro-N-piperidin-4-yl-D-phenylalanyl)-8-azabicyclo [3.2.1]oct-3-yl]-N-cyclohexyl-N',N'-diethylurea (composé n° 1)

### 1.1 : tert-butyl 3-(cyclohexylamino)-8-azabicyclo[3.2.1]octane-8-carboxylate

On dissout 9,1 g de cyclohexylamine et 10,3 g de Boc-nortropinone dans 150mL d'éthanol. On additionne à température ambiante 14,3 g de tétraisopropoxyde de titane. Le milieu réactionnel est agité pendant 16h. On additionne par fractions 2,6 g de borohydrure de sodium à 0°C, et le milieu réactionnel est alors agité à température ambiante durant 2 heures. Après addition de 50 ml d'eau et de 20 ml d'ammoniaque aqueux (28% dans l'eau), le précipité blanc formé dans le milieu réactionnel est filtré sur un lit de célite. Le solide est lavé plusieurs fois par le dichlorométhane. Les phases organiques sont ensuite rassemblées, lavées à l'eau, séchées sur MgSO₄ et concentrées. Le brut obtenu est chromatographié sur gel de silice en éluant avec un gradient de méthanol dans le dichlorométhane variant de 0% à 10%. On obtient 7,7g du composé endo *tert*-butyl 3-(cyclohexylamino)-8-azabicyclo[3.2.1]octane-8-carboxylate et 1,2 g du composé exo *tert*-butyl 3-(cyclohexylamino)-8-azabicyclo[3.2.1]octane-8-carboxylate. La suite de la synthèse concerne le composé endo.

### 1.2: tert-butyl 3-{cyclohexyl[(diethylamino)carbonyl]amino}-8-azabicyclo[3.2.1] octane-8-carboxylate

On place 0,64 mL de diphosgène dans 10 mL de dichlorométhane à 0°C sous N₂. Une solution de 1,0 g de composé endo *tert*-butyl 3-(cyclohexylamino)-8-azabicyclo[3.2.1]octane-8-carboxylate et de 2,26 mL de triéthylamine dans 10 mL de dichlorométhane est ajoutée goutte à goutte. La solution est agitée 30 min à 0°C puis 4h à température ambiante. On ajoute alors 5 mL de triéthylamine et 1,0 mL de diphosgène. Après agitation pendant 2h à température ambiante, 1,68 mL de diéthylamine sont ajoutés. Le mélange est agité à température ambiante pendant 16h. Après concentration, on ajoute de l'acide chlorhydrique 0,5N jusqu'à pH acide. On extrait au dichlorométhane jusqu'à épuisement de la phase aqueuse. La phase organique est lavée avec H₂O, puis avec une solution aqueuse saturée de chlorure de sodium. Après séchage sur MgSO₄, et concentration à sec, le résidu obtenu est chromatographié sur gel de silice en éluant avec un mélange 99/1 puis 95/5 de dichlorométhane et de méthanol pour conduire à 1,60 g de *tert*-butyl 3-{cyclohexyl[(diethylamino) carbonyl]amino}-8-azabicyclo[3.2.1] octane-8-carboxylate en mélange avec de la diéthylurée.

### 1.3 : N-8-azabicyclo[3.2.1]oct-3-yl-N-cyclohexyl-N',N'-diethylurea

On place 3,0 g de *tert*-butyl 3-{cyclohexyl[(diethylamino)carbonyl]amino}-8-azabicyclo[3.2.1]octane-8-carboxylate en mélange avec la diéthylurée dans 37 mL d'acide chlorhydrique 2N-dans le diéthyléther. Le milieu réactionnel est agité pendant 16h à température ambiante. Après évaporation à sec, on reprend avec une solution aqueuse d'acide chlorhydrique 1 N. On extrait à l'acétate d'éthyle puis on ajoute une solution aqueuse de soude 1N jusqu'à pH 10. On extrait à l'acétate d'éthyle jusqu'à épuisement de la phase aqueuse. La phase organique est lavée avec H₂O, puis avec une solution aqueuse saturée de chlorure de sodium. Après séchage sur MgSO₄, et concentration à sec, on obtient 1,8 g de *N*-8-azabicyclo[3.2.1]oct-3-yl]-*N*-cyclohexyl-*N'*,*N'*-diethylurea.

### 1.4: tert-butyl [(1R)-1-(4-chlorobenzyl)-2-(3-{cyclohexyl[(diethylamino)carbonyl] amino}-8-azabicyclo[3.2.1]oct-8-yl)-2-oxoethyl]carbamate

On solubilise 1,8 g de *N*-8-azabicyclo[3.2.1]oct-3-yl-*N*-cyclohexyl-*N*',*N*'-diethylurea dans 70 mL de dichlorométhane en présence de 1,75 g de Boc-D-4-chlorophénylalanine, de 0,79 g d'hydroxybenzotriazole, de 1,12 g de chlorhydrate de 1-(3-diméthylaminopropyl)-3-éthyl-carbodiimide et de 1,02 mL de diisopropyléthylamine. Le mélange est agité 16h à température ambiante. Après évaporation à sec, le résidu est hydrolysé et extrait à l'acétate d'éthyle jusqu'à épuisement de la phase aqueuse. La phase organique est lavée avec H₂O, puis avec une solution aqueuse saturée de chlorure de sodium. Après séchage sur MgSO₄ et concentration à sec, le brut est chromatographié sur gel de silice en éluant avec un mélange 99/1 de dichlorométhane et de méthanol pour conduire à 1,42 g de *tert*-butyl [(1*R*)-1-(4-chlorobenzyl)-2-(3-{cyclohexyl[(diethylamino)carbonyl]amino}-8-azabicyclo[3.2.1]oct-8-yl)-2-oxoethyl]carbamate.

### 1.5 : N-[8-(4-chloro-D-phenylalanyl)-8-azabicyclo[3.2.1]oct-3-yl]-N-cyclohexyl-N',N'-diethylurea

On place 1,42 g de *tert*-butyl [(1*R*)-1-(4-chlorobenzyl)-2-(3-{cyclohexyl [(diethylamino)carbonyl]amino}-8-azabicyclo[3.2.1]oct-8-yl)-2-oxoethyl]carbamate dans 12 mL d'acide chlorhydrique 2N dans le diéthyléther. Le milieu réactionnel est agité pendant 3h à température ambiante. On rajoute 5 mL d'acide chlorhydrique 2N dans le diéthyléther et l'agitation est maintenue 16h. Après évaporation à sec, le brut obtenu est chromatographié sur gel de silice en éluant avec un mélange 95/5/0,5 de dichlorométhane, de méthanol et d'ammoniaque aqueuse. On obtient 1,03 g de *N*-[8-(4-chloro-D-phenylalanyl)-8-azabicyclo[3.2.1 ]oct-3-yl]-*N*-cyclohexyl-*N*',*N*'-diethylurea.

### 1.6 : tert-butyl 4-{[(1R)-1-(4-chlorobenzyl)-2-(3-{cyclohexyl[(diethylamino)carbonyl] amino}-8-azabicyc)o[3.2.1]oct-8-yl)-2-oxoethyl]amino}piperidine-1-carboxylate

On solubilise 0,41 g de *N*-[8-(4-chloro-D-phenylalanyl)-8-azabicyclo[3.2.1]oct-3-yl]-*N*-cyclohexyl-*N'*,*N'*-diethylurea dans 4 mL de dichlorométhane sous N₂ en présence de 0,17 g de *N*-BOC-pipéridone et de 0,23 g de triacétoxyborohydrure de sodium et on agite pendant 16h à température ambiante. Après évaporation et hydrolyse, on extrait à l'acétate d'éthyle jusqu'à épuisement de la phase aqueuse. La phase organique est lavée avec H₂O, puis avec une solution aqueuse saturée de chlorure de sodium. On sèche sur MgSO₄ et on concentre à sec. Le brut est chromatographié sur gel de silice en éluant avec mélange 98/2 de dichlorométhane et de méthanol. On obtient alors 0,37 g de *tert-*butyl 4-{[(1*R*)-1-(4-chlorobenzyl)-2-(3-{cyclohexyl[(diethylamino)carbonyl]amino}-8-azabicyclo[3.2.1]oct-8-yl)-2-oxoethyl]amino}piperidine-1-carboxylate.

### 1.7: chlorhydrate de N-[8-(4-chloro-N-piperidin-4-yl-D-phenylalanyl)-8-azabicyclo [3.2.1]oct-3-yl]-N-cyclohexyl-N',N'-diethylurea

On solubilise 0,37 g de *tert*-butyl 4-{[(1*R*)-1-(4-chlorobenzyl)-2-(3-{cyclohexyl[(diethylamino)carbonyl]amino}-8-azabicyclo[3.2.1]oct-8-yl)-2-oxoethyl]amino}piperidine-1-carboxylate dans 2,74 mL d'acide chlorhydrique 2N dans le diéthyléther. Le milieu réactionnel est agité pendant 16h à température ambiante. Après évaporation à sec, le brut est chromatographié sur gel de silice en éluant avec un mélange 95/5/0,5 de dichlorométhane, de méthanol et d'ammoniaque aqueuse. On obtient alors 0,27 g de chlorhydrate de *N*-[8-(4-chloro-*N*-piperidin-4-yl-D-phenylalanyl)-8-azabicyclo[3.2.1]oct-3-yl]-*N*-cyclohexyl-*N'*,*N*-diethylurea.
Point de fusion > 210°C, M+H⁺ = 572, [α]_{D}²⁰ = -2,0 (c=1,002g/100 mL, MeOH).

### Exemple 2 : N-{8-[4-chloro-N-(tetrahydro-2H-pyran-4-yl)-D-phenylalanyl]-8-azabicyclo[3.2.1]oct-3-yl}-N-cyclohexyl-N',N'-diethylurea (composé n° 4)

### 2.1 : N-{8-[4-chloro-N-(tetrahydro-2H-pyran-4-yl)-D-phenylalanyl]-8-azabicyclo [3.2.1]oct-3-yl}-N-cyclohexyl-N',N'-diethylurea

On solubilise 0,30 g du composé endo *N*-[8-(4-chloro-D-phenylalanyl)-8-azabicyclo[3.2.1]oct-3-yl]-*N*-cyclohexyl-*N*',*N*'-diethylurea obtenu lors de l'étape 1.5 dans 3 mL de dichlorométhane sous N₂ en présence de 0,061 g de tétrahydro-4*H*-pyran-4-one et de 0,17 g de triacétoxyborohydrure de sodium. Le milieu réactionnel est agité pendant 3 jours à température ambiante. Après évaporation et hydrolyse avec une solution aqueuse saturée d'hydrogénocarbonate de sodium, on extrait à l'acétate d'éthyle jusqu'à épuisement de la phase aqueuse. La phase organique est lavée avec H₂O, puis avec une solution aqueuse saturée de chlorure de sodium. On sèche sur MgSO₄ et on concentre à sec. Le brut est chromatographié sur gel de silice en éluant avec un mélange 99/1 puis 98/2 de dichlorométhane et de méthanol. On obtient alors 0,23 g de *N*-{8-[4-chloro-*N-*(tetrahydro-2*H-*pyran-4-yl)-D-phenylalanyl]-8-azabicyclo[3.2.1]oct-3-yl}-*N*-cyclohexyl-*N'*,*N'*-diethylurea.

### 2.2 : Chlorhydrate de N-{8-[4-chloro-N-(tetrahydro-2H-pyran-4-yl)-D-phenylalanyl]-8-azabicyclo[3.2.1]oct-3-yl}-N-cyclohexyl-N,N'-diethylurea

On solubilise 0,23 g de *N*-{8-[4-chloro-*N*-(tetrahydro-2*H*-pyran-4-yl)-D-phenylalanyl]-8-azabicyclo[3.2.1]oct-3-yl}-*N*-cyclohexyl-*N'*,*N'*-diethylurea dans 5 mL de diéthyléther puis on ajoute 0,20 mL d'acide chlorhydrique 2N dans le diéthyléther. On triture, on rince avec du diéthyléther et on essore le précipité obtenu. Les cristaux sont ensuite séchés sur P₂O₅ sous pression réduite. On obtient 0,19 g de *N*-{8-[4-chloro-*N-*(tetrahydro-2*H*-pyran-4-yl)-D-phehylalanyl]-8-azabicyclo[3.2.1]oct-3-yl}-*N*-cyclohexyl-*N'*,*N'*-diethylurea.
Point de fusion > 210°C, M+H⁺ = 573, [α]_{D}²⁰ = -5,9 (c=0,553g/100 mL, DMSO). RMN ¹H (200MHz, DMSO-d + D₂O) : 7,31 (d, J=8 Hz, 2H), 7,19 (d, J=8 Hz, 2H), 4,62 (m, 1 H), 4,41 (m, 1 H), 3,90 (m, 3H) 3,25 (m, 5H), 2,88 (m, 5H), 2,10-1,05 (m, 23H), 0,91 (t, J=4Hz, 6H). Analyse élémentaire : exp %C= 61,46, %H : 8,10, %N : 8,88 ; th : %61,50, %H : 8,34 ,%N : 8,96

### Exemple 3 : N-[8-(N-8-azabicyclo[3.2.1]oct-3-yl-4-chloro-D-phenylalanyl)-8-azabicyclo[3.2.1]oct-3-yl]-N-cyclohexyl-N',N'-diethylurea (composé n° 5)

### 3.1 : tert-butyl 3-{[(1R)-1-(4-chlorobenzyl)-2-(3-{cyclohexyl[(diethylamino)carbonyl] amino}-8-azabicyclo[3.2.1]oct-8-yl)-2-oxoethyl]amino}-8-azabicyclo[3.2.1]octane-8-carboxylate

On solubilise 0,24 g du composé endo *N*-[8-(4-chloro-D-phenylalanyl)-8-azabicyclo[3.2.1]oct-3-yl]-*N*-cyclohexyl-*N'*,*N'*-diethylurea obtenu lors de l'étape 1.5 dans 5 mL de dichlorométhane sous N₂ en présence de 0,169 g de Boc-nortropanone et de 0,26 g de triacétoxyborohydrure de sodium. Le milieu réactionnel est agité pendant 16h à température ambiante. On ajoute 0,085 g de Boc-nortropinone et 2,6g de triacétoxyborohydrure de sodium et l'agitation est maintenue 2 jours. Après évaporation à sec, on ajoute 1 mL de méthanol et 4 g de résine DOWEX^{®} 50X2. On agite pendant 1h30. Après filtration et lavage de la résine par du tétrahydrofurane et du méthanol, on relargue le composé attendu en ajoutant une solution 1 N d'ammoniaque dans le méthanol. Le méthanol est évaporé pour conduire à 0,21 g de endo et exo *tert*-butyl 3-{[(1*R*)-1-(4-chlorobenzyl)-2-(3-{cyclohexyl[(diethylamino)carbonyl]amino}-8-azabicyclo[3.2.1]oct-8-yl)-2-oxoethyl]amino}-8-azabicyclo[3.2.1]octane-8-carboxylate. La suite de la synthèse se fait sur ce mélange.

### 3.2 : Chlorhydrate de N-[8-(N-8-azabicyclo[3.2.1]oct-3-yl-4-chloro-D-phenylalanyl)-8-azabicyclo[3.2.1]oct-3-yl]-N-cyclohexyl-N',N'-diethylurea

On place 0,21 g de *tert*-butyl 3-{[(1*R*)-1-(4-chlorobenzyl)-2-(3-{cyclohexyl [(diethylamino)carbonyl]amino}-8-azabicyclo[3.2.1]oct-8-yl)-2-oxoethyl]amino}-8-azabicyclo[3.2.1]octane-8-carboxylate dans 1,53 mL d'acide chlorhydrique 2N dans le diéthyléther. Le milieu réactionnel est agité une nuit à température ambiante. On triture, on rince avec du diéthyléther et on essore le précipité obtenu. Les cristaux sont ensuite séchés sur P₂O₅ sous pression réduite. On obtient 0,12g de chlorhydrate de *N*-[8-(*N*-8-azabicyclo[3.2.1]oct-3-yl-4-chloro-D-phenylalanyl)-8-azabicyclo[3.2.1]oct-3-yl]-*N-*cyclohexyl-*N*',*N*'-diethylurea.
Point de fusion = 155°C; M+H⁺ = 598.

### Exemple 4 : N-[8-(4-chloro-N-{[(2S,4R)-4-hydroxypyrrolidin-2-yl]methyl}-D-phenylalanyl)-8-azabicyclo[3.2.1]oct-3-yl]-N-cyclohexyl-N',N'-diethylurea (composé n°11)

### 4.1 : tert-butyl (2S,4R)-4-hydroxy-2-{[methoxy(methyl)amino]carbonyl}pyrrolidine-1-carboxylate

On dissout 1,85 g de (4*R*)-1-(*tert*-butoxycarbonyl)-4-hydroxy-L-proline dans 80 mL de dichlorométhane à 0°C sous N₂. On ajoute 3,72 mL de triéthylamine et 1,97 mL de chloroformiate de *tert*-butyle. Le milieu réactionnel est agité pendant 1h à température ambiante. Parallèlement, une solution de diméthylhydroxylamine est préparée par addition de 1,86 mL de triéthylamine à une solution de 1,56 g de chlorhydrate de diméthylhydroxylamine dans le dichlorméthane, puis par filtration du chlorhydrate de triéthylamine. Cette seconde solution est ajoutée doucement à 0°C à la première. Le milieu réactionnel est agité pendant 16h à température ambiante. Après hydrolyse avec une solution aqueuse d'acide chlorhydrique 0,5N, on extrait au dichlorométhane jusqu'à épuisement de la phase aqueuse. La phase organique est séchée sur MgSO₄ et concentrée à sec. On obtient 0,9 g de *tert*-butyl (2*S*,4*R*)-4-hydroxy-2-{[methoxy(methyl) amino]carbonyl}pyrrolidine-1-carboxylate qui est utilisé tel quel par la suite.

### 4.2 : tert-butyl (2S,4R)-2-formyl-4-hydroxypyrrolidine-1-carboxylate

On dissout 0,9 g de *tert*-butyl (2*S*,4*R*)-4-hydroxy-2-{[methoxy(methyl)amino] carbonyl}pyrrolidine-1-carboxylate dans 31 mL de diéthyléther sous N₂. On place le milieu réactionnel à 0°C et on ajoute doucement 3,41 mL d'hydrure d'hydrure de lithium aluminium 1 N dans le tétrahydrofurane. Le mélange est agité pendant 2h à 0°C. Après hydrolyse avec une solution aqueuse de sulfate de potassium, on extrait au diéthyléther jusqu'à épuisement de la phase aqueuse. On sèche sur MgSO₄ et on concentre à sec. Le brut obtenu est chromatographié sur gel de silice en éluant avec un mélange 9/1 de dichlorométhane et de méthanol. On obtient 0,45g de *tert*-butyl (2*S*,4*R*)-2-formyl-4-hydroxypyrrolidine-1-carboxylate.

### 4.3 : tert-butyl (2S,4R)-2-({[(1R)-1-(4-chlorobenzyl)-2-(3-{cyclohexyl[(diethylamino) carbonyl]amino}-8-azabicyclo[3.2.1]oct-8-yl)-2-oxoethyl]amino}methyl)-4-hydroxypyrrolidine-1-carboxylate

On solubilise 0,24 g du composé endo *N*-[8-(4-chloro-D-phenylalanyl)-8-azabicyclo[3.2.1]oct-3-yl]-*N*-cyclohexyl-*N*',*N*'-diethylurea obtenu lors de l'étape 1.5 dans 3 mL de dichlorométhane sous N₂ en présence de 0,16 g de *tert*-butyl (2*S*,4*R*)-2-formyl-4-hydroxypyrrolidine-1-carboxylate et de 0,233 g de triacétoxyborohydrure de sodium. Le milieu réactionnel est agité pendant 16h à température ambiante. Après évaporation à sec, on ajoute 1 mL de méthanol et 4 g de résine DOWEX^{®} 50X2. On agite pendant 1h30. Après filtration et lavage de la résine par du tétrahydrofurane et du méthanol, on relargue le composé attendu en ajoutant une solution 1 N d'ammoniaque dans le méthanol: Le méthanol est évaporé pour conduire à 0,28 g de *tert*-butyl (2*S*,4*R*)-2-({[(1*R*)-1-(4-chlorobenzyl)-2-(3-{cyclohexyl[(diethylamino)carbonyl]amino}-8-azabicyclo[3.2.1]oct-8-yl)-2-oxoethyl]amino}methyl)-4-hydroxypyrrolidine-1-carboxylate.

### 4.4: N-[8-(4-chloro-N-{[(2S,4R)-4-hydroxypyrrolidin-2-yl]methyl}-D-phenylalanyl)-8-azabicyclo[3.2.1]oct-3-yl]-N-cyclohexyl-N',N'-diethylurea

On solubilise 0,28 g de *tert*-butyl (2*S*,4*R*)-2-({[(1*R*)-1-(4-chlorobenzyl)-2-(3-{cyclohexyl[(diethylamino)carbonyl]amino}-8-azabicyclo[3.2.1]oct-8-yl)-2-oxoethyl]amino}methyl)-4-hydroxypyrrolidine-1-carboxylate dans 5 mL de diéthyléther puis on ajoute 2,7 mL d'acide chlorhydrique 2N dans le diéthyléther. On triture, on rince avec du diéthyléther et on essore le précipité obtenu qui est chromatographié sur gel de silice en éluant avec un mélange 90/10/1 de dichlorométhane, de méthanol et d'ammoniaque aqueuse.. On obtient 0,18 g de *N*-[8-(4-chloro-*N*-{[(2*S*,4*R*)-4-hydroxypyrrolidin-2-yl]methyl}-D-phenylalanyl)-8-azabicyclo[3.2.1]oct-3-yl]-*N*-cyclohexyl-*N*',*N*'-diethylurea.

### 4.5 : Chlorhydrate de N-[8-(4-chloro-N-{[(2S,4R)-4-hydroxypyrrolidin-2-yl]methyl}-D-phenylalanyl)-8-azabicyclo[3.2.1]oct-3-yl]-N-cyclohexyl-N',N'-diethylurea

On place 0,18 g de *N*-[8-(4-chloro-*N*-{[(2*S*,4*R*)-4-hydroxypyrrolidin-2-yl]methyl}-D-phenylalanyl)-8-azabicyclo[3.2.1]oct-3-yl]-*N-*cyclohexyl-*N'*,*N'*-diethylurea dans 0,4 mL d'acide chlorhydrique 2N dans le diéthyléther. On triture, on rince avec du diéthyléther et on essore le précipité obtenu. On obtient 0,16 g de chlorhydrate de *N*-[8-(4-chloro-*N-*{[(2*S*,4*R*)-4-hydroxypyrrolidin-2-yl]methyl}-D-phenylalanyl)-8-azabicyclo[3.2.1]oct-3-yl]-*N-*cyclohexyl-*N*',*N*'-diethylurea.
Point de fusion > 200°C, M+H⁺ = 588 , [α]_{D}²⁰ = -10,7° (c=0,646 g/100 mL, DMSO).

### Exemple 5 : N-{8-[N-(2-aminoethyl)-4-chloro-D-phenylalanyl]-8-azabicyclo [3.2.1]oct-3-yl}-N-cyclohexyl-N',N'-diethylurea (composé n° 18)

### 5.1 : tert-butyl (2-{[(1R)-1-(4-chlorobenzyl)-2-(3-{cyclohexyl[(diethylamino) carbonyl]amino}-8-azabicyclo[3.2.1]oct-8-yl)-2-oxoethyl]amino}ethyl)carbamate

On solubilise 0,24 g du composé endo *N*-[8-(4-chloro-D-phenylalanyl)-8-azabicyclo[3.2.1]oct-3-yl]-*N*-cyclohexyl-*N*',*N*'-diethylurea obtenu lors de l'étape 1.5 dans 5 mL de dichlorométhane sous N₂ en présence de 0,10 g de *tert*-butyl N-(2-oxoethyl)carbamate et de 0,22 g de triacétoxyborohydrure de sodium. Le milieu réactionnel est agité pendant 16h à température ambiante. Après évaporation à sec, on ajoute 0,05 g de *tert*-butyl N-(2-oxoethyl)carbamate et 0,1g de triacétoxyborohydrure de sodium et on maintient l'agitation pendant 3 jours. Après addition de 1 mL de méthanol et 4 g de résine DOWEX^{®} 50X2, on agite pendant 1h30. Après filtration et lavage de la résine par du tétrahydrofurane et du méthanol, on relargue le composé attendu en ajoutant une solution 1 N d'ammoniaque dans le méthanol. Le méthanol est évaporé pour conduire à 0,14 g de *tert*-butyl (2-{[(1*R*)-1-(4-chlorobenzyl)-2-(3-{cyclohexyl [(diethylamino)carbonyl]amino}-8-azabicyclo[3.2.1]oct-8-yl)-2-oxoethyl]amino}ethyl) carbamate.

### 5.2 : N-{8-[N-(2-aminoethyl)-4-chloro-D-phenylalanyl]-8-azabicyclo[3.2.1]oct-3-yl}-N-cyclohexyl-N',N'-diethylurea

On place 0,14 g de *tert*-butyl (2-{[(1*R*)-1-(4-chlorobenzyl)-2-(3-{cyclohexyl [(diethylamino)carbonyl]amino}-8-azabicyclo[3.2.1]oct-8-yl)-2-oxoethyl]amino}ethyl)carbamate dans 1,1 mL d'acide chlorhydrique 2N dans le diéthyléther. Le milieu réactionnel est agité 1h à température ambiante. On triture, on rince avec du diéthyléther et on essore le précipité obtenu. Le sel ainsi obtenu est chromatographié sur gel de silice en éluant avec un mélange 98/2/0,2 puis 95/5/0,5 de dichlorométhane, de méthanol et d'ammoniaque aqueuse. On obtient 0,05g de chlorhydrate de *N*-{8-[*N*-(2-aminoethyl)-4-chloro-D-phenylalanyl]-8-azabicyclo[3.2.1]oct-3-yl}-*N*-cyclohexyl-*N*',*N*'-diethylurea.
Point de fusion = 105°C, M+H⁺ = 532, [α]_{D}²⁰ = -12,6°(c=0,521 g/100 mL, DMSO).

### Exemple 6 : N-(8-{4-chloro-N-[(3R)-1,2,3,4-tetrahydroisoquinolin-3-ylmethyl]-D-phenylalanyl}-8-azabicyclo[3.2.1]oct-3-yl)-N-cyclohexyl-N',N'-diethylurea (composé n° 22)

### 6.1 : tert-butyl (3R)-3-{[methoxy(methyl)amino]carbonyl}-3,4-dihydroisoquinoline-2(1H)-carboxylate

On dissout 6,95 g de Boc-D-Tic-OH dans 25 mL de dichlorométhane. On ajoute 2,69 g de chloryhydrate de diméthylhydroxylamine et 11,68 g de bromo-tris-pyrrolidino-phosphonium hexafluorophosphate. Après refroidissement de la solution à 0°C, on additionne lentement 9,72 mL de diisopropylamine. Puis le milieu réactionnel est agité à température ambiante pendant .16h. Après hydrolyse, on extrait à l'acétate d'éthyle jusqu'à épuisement de la phase aqueuse. On sèche sur MgSO₄ et on concentre à sec. Le brut obtenu est chromatographié sur gel de silice en éluant avec un mélange 99/1 de dichlorométhane et de méthanol. On obtient 7,4 g de *tert*-butyl (3R)-3-{[methoxy(methyl)amino]carbonyl}-3,4-dihydroisoquinoline-2(1*H*)-carboxylate.

### 6.2 : tert-butyl (3R)-3-formyl-3,4-dihydroisoquinoline-2(1H)-carboxylate

On dissout 7,4g de *tert*-butyl (3*R*)-3-{[methoxy(methyl)amino]carbonyl}-3,4-dihydroisoquinoline-2(1*H*)-carboxylate dans 31 mL de diéthyléther sous N₂. On place le milieu réactionnel à 0°C et on ajoute doucement 0,98 g d'hydrure d'hydrure de lithium aluminium. Le mélange est agité pendant 45 min à 0°C. Après hydrolyse avec une solution aqueuse de sulfate de potassium, on extrait au diéthyléther jusqu'à épuisement de la phase aqueuse. Les phases organiques sont lavées avec une solution aqueuse d'acide chlorhydrique 3N, avec une solution aqueuse saturée d'hydrogénocarbonate de sodium, puis avec H₂O et enfin avec une solution aqueuse saturée de chlorure de sodium. On sèche sur MgSO₄ et on concentre à sec. On obtient 3,5 g de *tert*-butyl (3*R*)-3-formyl-3,4-dihydroisoquinoline-2(1*H*)-carboxylate qui est utilisé tel quel par la suite.

### 6.3 : tert-butyl (3R)-3-({[(1R)-1-(4-chlorobenzyl)-2-methoxy-2-oxoethyl]amino} methyl)-3,4-dihydroisoquinoline-2(1H)-carboxylate

On solubilise 3,5 g de *tert*-butyl (3*R*)-3-formyl-3,4-dihydroisoquinoline-2(1*H*)-carboxylate dans 67 mL de dichlorométhane sous N₂ en présence de 4,02 g d'ester méthylique de la D-4-chlorophenyl alanine et de 3,69 g de triacétoxyborohydrure de sodium. Le milieu réactionnel est agité pendant 16h à température ambiante. Après hydrolyse, on extrait dichlorométhane jusqu'à épuisement de la phase aqueuse. On sèche sur MgSO₄ et on concentre à sec. Le brut est chromatographié sur gel de silice en éluant avec un gradient de méthanol dans le dichlorométhane variant de1 % à 3%. On obtient alors 6,1 g de *tert*-butyl (3*R*)-3-({[(1*R*)-1-(4-chlorobenzyl)-2-methoxy-2-oxoethyl]amino}methyl)-3,4-dihydroisoquinoline-2(1*H*)-carboxylate.

### 6.4 : N-{[(3R)-2-(tert-butoxycarbonyl)-1,2,3,4-tetrahydroisoquinolin-3-yl]methyl}-4-chloro-D-phenylalanine

On solubilise 6,4 g de *tert*-butyl (3*R*)-3-({[(1*R*)-1-(4-chlorobenzyl)-2-methoxy-2-oxoethyl]amino}methyl)-3,4-dihydroisoquinoline-2(1*H*)-carboxylate dans 150 mL d'un mélange tétrahydrofurane/eau/MeoH (1/1/1) à 0°C et on ajoute 0,99 g d'hydroxyde de lithium hydrate. L'agitation est maintenue 3h à 0°C. on ajoute alors 0,5 g d'hydroxyde de lithium hydrate. Le milieu est maintenu à 0°C pendant 16h. On ajoute du sulfate, de potassium jusqu'à pH 7. Le précipité obtenu est essoré et rincé au diéthyléther. Après séchage sur P₂O₅, on obtient 7,2 g de *N-*{[(3*R*)-2-(*tert*-butoxycarbonyl)-1,2,3,4-tetrahydroisoquinolin-3-yl]methyl}-4-chloro-D-phenylalanine.

### 6.5: tert-butyl (3R)-3-({[(1R)-1-(4-chlorobenzyl)-2-(3-{cyclohexyl[(diethylamino) carbonyl]amino}-8-azabicyclo[3.2.1]oct-8-yl)-2-oxoethyl]amino}methyl)-3,4-dihydroisoquinoline-2(1H)-carboxylate

On solubilise 0,2 g de composé endo *N*-8-azabicyclo[3.2.1]oct-3-yl-*N*-cyclohexyl-*N*',*N*'-diethylurea obtenu à l'étape 1.3 dans 3,25 mL de dichlorométhane en présence de 0,35 g de *N*-{[(3R)-2-(*tert*-butoxycarbonyl)-1,2,3,4-tetrahydroisoquinolin-3-yl]methyl}-4-chloro-D-phenylalanine obtenu à l'étape 6.5, de 0,088 g d'hydroxybenzotriazole, de 0,125 g de chlorhydrate de 1-(3-diméthylaminopropyl)-3-éthyl-carbodiimide et de 0,17 mL de diisopropyléthylamine. Le mélange est agité 16h à température ambiante. Après évaporation à sec, le résidu est hydrolysé et extrait à l'acétate d'éthyle jusqu'à épuisement de la phase aqueuse. Après séchage sur MgSO₄ et concentration à sec, le brut est chromatographié sur gel de silice en éluant avec un gradient de méthanol dans le dichlorométhane variant de 0% à 3%. On obtient 0,29 g de *tert*-butyl (3*R*)-3-({[(1*R*)-1-(4-chlorobenzyl)-2-(3-{cyclohexyl[(diethylamino)carbonyl]amino}-8-azabicyclo[3.2.1]oct-8-yl)-2-oxoethyl]amino}methyl)-3,4-dihydroisoquinoline-2(1*H*)-carboxylate.

### 6.6 : N-(8-{4-chloro-N-[(3R)-1,2,3,4-tetrahydroisoquinolin-3-ylmethyl]-D-phenylalanyl}-8-azabicyclo[3.2.1]oct-3-yl)-N-cyclohexyl-N',N'-diethylurea

On place 0,29 g de *tert-*butyl (3*R*)-3-({[(1*R*)-1-(4-chlorobenzyl)-2-(3-{cyclohexyl[(diethylamino)carbonyl]amino}-8-azabicyclo[3.2.1]oct-8-yl)-2-oxoethyl]amino} methyl)-3,4-dihydroisoquinoline-2(1*H*)-carboxylate dans 1,1 mL d'acide chlorhydrique 4N dans le dioxane. Le milieu réactionnel est agité 1h à température ambiante. Après hydrolyse avec une solution aqueuse de soude 1N, on extrait au dichlorométhane jusqu'à épuisement de la phase aqueuse. On sèche sur MgSO₄ et on concentre à sec. Le brut obtenu est chromatographié sur gel de silice en éluant avec un mélange 98/2 de dichlorométhane et de méthanol. On obtient 0,12 g de *N*-(8-{4-chloro-*N*-[(3*R*)-1,2,3,4-tetrahydroisoquinolin-3-ylmethyl]-D-phenylalanyl}-8-azabicyclo[3.2.1]oct-3-yl)-*N-*cyclohexyl-*N*',*N*'-diethylurea.

### 6.7: Chlorhydrate de N-(8-{4-chloro-N-[(3R)-1,2,3,4-tetrahydroisoquinolin-3-ylmethyl]-D-phenylalanyl}-8-azabicyclo[3.2.1]oct-3-yl)-N-cyclohexyl-N',N'-diethylurea

On dissout 0,12 g de *N*-(8-{4-chloro-*N-*[(3*R*)-1,2,3,4-tetrahydroisoquinolin-3-ylmethyl]-D-phenylalanyl}-8-azabicyclo[3.2.1]oct-3-yl)-*N-*cyclohexyl-*N*',*N*'-diethylurea dans 5 mL de dichlorométhane et on ajoute 0,4 mL d'acide chlorhydrique 4N dans le dioxane. Après évaporation, on reprend le résidu dans le diéthyléther, on rince avec du diéthyléther et on essore le précipité obtenu. On obtient 0,12 g de chlorhydrate de *N*-(8-{4-chloro-*N*-[(3*R*)-1,2,3,4-tetrahydroisoquinolin-3-ylmethyl]-D-phenylalanyl}-8-azabicyclo [3.2.1]oct-3-yl)-*N*-cyclohexyl-*N*',*N*'-diethylurea.
Point de fusion = 182°C, M+H⁺ = 634 , [α]_{D}²⁰ =-10,2° (c=0,857 g/100 mL, DMSO).

### Exemple 7 : N-{8-[4-chloro-N-(pyridin-2-ylmethyl)-D-phenylalanyl]-8-azabicyclo[3.2.1]oct-3-yl}-N-cyclohexyl-N',N'-diethylurea (composé n°23)

### 7.1 : N-{8-[4-chloro-N-(pyridin-2-ylmethyl)-D-phenylalanyl]-8-azabicyclo[3.2.1]oct-3-yl}-N-cyclohexyl-N',N'-diethylurea

On solubilise 0,24 g de *N*-[8-(4-chloro-D-phenylalanyl)-8-azabicyclo[3.2.1]oct-3-yl]-*N*-cyclohexyl-*N*',*N*'-diethylurea obtenu lors de l'étape 1.5 dans 3 mL de dichlorométhane, sous N₂ en présence de 0,05 mL de 2-pyridine carboxaldehyde et de 0,22 g de triacétoxyborohydrure de sodium. Le milieu réactionnel est agité pendant 16h à température ambiante. Après évaporation à sec et addition de 1 mL de méthanol et 4 g de résine DOWEX^{®} 50X2, on agite pendant 1h30. Après filtration et lavage de la résine par du tétrahydrofurane et du méthanol, on relargue le composé attendu en ajoutant une solution 1 N d'ammoniaque dans le méthanol. Le méthanol est évaporé pour conduire à 0,054 g de *N-*{8-[4-chloro-*N*-(pyridin-2-ylmethyl)-D-phenylalanyl]-8-azabicyclo[3.2.1]oct-3-yl}-*N*-cyclohexyl-*N*',*N*'-diethylurea.

### 7.2: Chlorhydrate de N-{8-[4-chloro-N-(pyridin-2-ylmethyl)-D-phenylalanyl]-8-azabicyclo[3.2.1]oct-3-yl}-N-cyclohexyl-N',N'-diethylurea

On place 0,054 g de dans 2 mL d'isopropanol et on ajoute 0,06 mL d'acide chlorhydrique 2N dans le déthyléther. Après évaporation, on reprend le résidu dans le diéthyléther , on rince avec du diéthyléther et on essore le précipité obtenu. On obtient 0,045 g de chlorhydrate de *N*-{8-[4-chloro-*N*-(pyridin-2-ylmethyl)-D-phenylalanyl]-8-azabicyclo[3.2.1]oct-3'-yl}-*N*-cyclohexyl-*N'*,*N'*-diethylurea.
Point de fusion = 119°C, M+H⁺ = 580.

Le tableau qui suit illustre les structures chimiques et les propriétés physiques de quelques exemples de composés selon l'invention, à savoir des composés de formule (I bis), correspondant à des composés de formule (I) dans lesquels Rₐ = R_{a'} = R₅ = H et R₃ représente un atome de chlore situé en position para sur le noyau phényle auquel il est rattaché. Dans ce tableau :
- dans la colonne « sel », « HCl» représente un composé sous forme de chlorhydrate,
- « PF » représente le point de fusion mesuré pour le composé,
- Me et Et représentent respectivement des groupes méthyle et éthyle.

**Tableau**

| | | | | | |
|---|---|---|---|---|---|
| 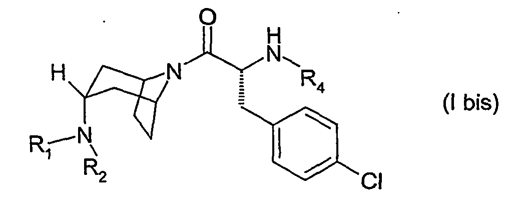 | | | | | |

| **N°** | **R₁** | **R₂** | **R₄** | **Sel** | **PF (°C)** |
|---|---|---|---|---|---|
| **1** | cyclohexyl | -CO-N(Et)₂ | 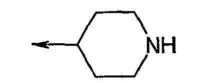 | HCl | >210 |
| **2** | cyclohexyl | -CO-N(Et)₂ | 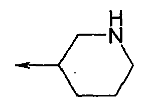 | HCl | 162 |
| **3** | cyclohexyl | -CO-N(Et)₂ | 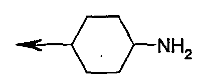 | HCl | 208 |
| **4** | cyclohexyl | -CO-N(Et)₂ | 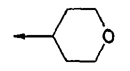 | HCl | >210 |
| **5** | cyclohexyl | -CO-N(Et)₂ | 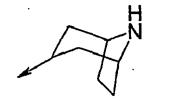 | HCl | 155 |
| **6** | cyclohexyl | -CO-N(Et)₂ | 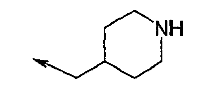 | HCl | 171 |
| **7** | cyclohexyl | -CO-N(Et)₂ | 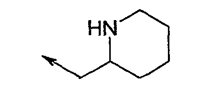 | HCl | 166 |
| **8** | cyclohexyl | -CO-N(Et)₂ | 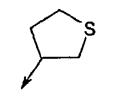 | HCl | >200 |
| **9** | cyclohexyl | -CO-N(Et)₂ | 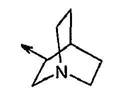 | HCl | 192 |
| **10** | cyclohexyl | -CO-N(Et)₂ | 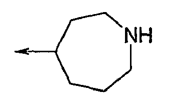 | HCl | 159 |
| **11** | cyclohexyl | -CO-N(Et)₂ | 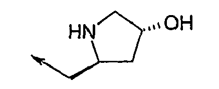 | HCl | >200 |
| **12** | cyclohexyl | -CO-N(Et)₂ | 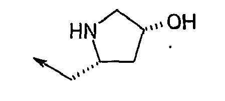 | CF₃CO₂H | 90 |
| **13** | cyclohexyl | -CO-N(Et)₂ | 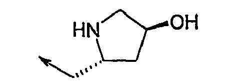 | HCl | |
| **14** | cyclohexyl | -CO-N(Et)₂ | 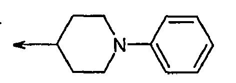 | HCl | 152 |
| **15** | cyclohexyl | -CO-N(Et)₂ | 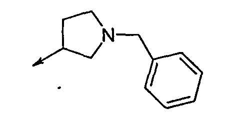 | HCl | 162 |
| **16** | cyclohexyl | -CO-N(Et)₂ | 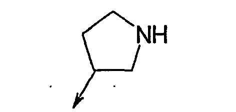 | HCl | 220 |
| **17** | cyclohexyl | -CO-N(Et)₂ | 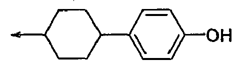 | HCl | 176 |
| **18** | cyclohexyl | -CO-N(Et)₂ | 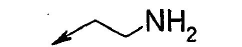 | HCl | 105 |
| **19** | cyclohexyl | -CO-N(Et)₂ | 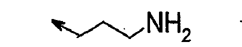 | HCl | 215 |
| **20** | cyclohexyl | -CO-N(Et)₂ | 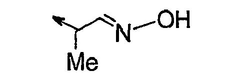 | HCl | 132 |
| **21** | cyclohexyl | -CO-N(Et)₂ | 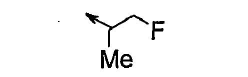 | HCl | >200 |
| **22** | cyclohexyl | -CO-N(Et)₂ | 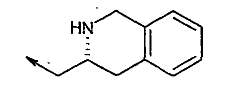 | HCl | 182 |
| **23** | cyclohexyl | -CO-N(Et)₂ | 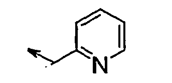 | HCl | 119 |
| **24** | cyclohexyl | -CO-N(Et)₂ | 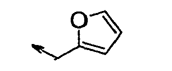 | HCl | >200 |
| **25** | cyclohexyl | -CO-N(Et)₂ | 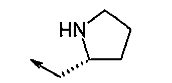 | HCl | |
| **26** | cyclohexyl | -CO-N(Et)₂ | 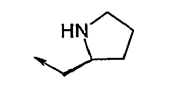 | HCl | |
| **27** | cyclohexyl | -CO-N(Et)₂ | 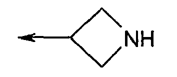 | HCl | 170 |
| **28** | cyclohexyl | -CO-N(Et)₂ | 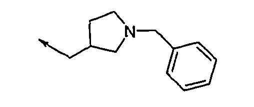 | HCl | 158 |

Les composés selon l'invention ont fait l'objet d'essais pharmacologiques permettant de déterminer leur effet agoniste des récepteurs aux mélanocortines, en particulier leur effet agoniste du récepteur MC3 et/ou MC4.

### Evaluation de l'affinité des composés de formule (I) selon l'invention envers les récepteurs MC3 et MC4

Ce test d'affinité est réalisé par la mesure de la liaison du [¹²⁵I]-[Nle⁴-D-Phe⁷]-α-MSH aux membranes cellulaires : le déplacement de ce radio-ligand est utilisé pour identifier des inhibiteurs de la liaison spécifique aux récepteurs recombinants mélanocortine.

Pour ce test, on utilise des membranes préparées à partir des cellules CHO-K1 exprimant le récepteur humain MC4 à forte densité (Euroscreen) ou des membranes achetées (Perkin Elmer Life Sciences, Receptor Biology) des cellules HEK-293 exprimant des récepteurs hMC3. Les cellules CHO-K1 transfectées avec le gène du récepteur hMC4 (Euroscreen) sont ensemencées dans le milieu de culture DMEM/Nutrient Mix F12 contenant 10% sérum de veau (Biowhittaker), 1% pyruvate de sodium, 1% L-glutamine, 1% acides aminés non-essentiels, 0.4 mg/ml généticine (G418) et 0.5% PenStrep, ces produits étant fournis par Gibco/BRI, sauf le sérum de veau. Les cellules sont grattées à 80% de confluence et les culots de cellules sont congelés à -80°C.

Un tube de cellules (environ 70 x 10⁶ cellules) est décongelé sur glace et resuspendu dans 10 ml de tampon de binding [25 mM HEPES, pH 7.0, 1 mM MgCl₂, 1.5 mM CaCl₂, 100 mM NaCl, 1 mM 1,10-phenanthroline et 1 tablette de Complete^{TR} (inhibiteur de protéases de Roche) dans 50 ml de tampon] par polytronnage 20 s. La suspension est centrifugée 20 min à 19500 t/min à 4°C. Le surnageant est jeté et le culot est resuspendu dans 5 ml tampon de binding. Par un test de Bradford, on dose la quantité de protéines présentes dans l'échantillon et on ajuste la concentration à 3 µg/25 µl par dilution dans du tampon de binding. [¹²⁵I]-[Nle⁴, D-Phe⁷]-α-MSH est dilué dans du tampon de binding + 0.2% BSA. Des billes SPA (wheatgerm agglutinine polyvinyltoluene, Amersham Pharmacia Biotech), sont hydratées dans le tampon de binding + 0.2% BSA et ensuite mélangées avec l'homogénat de cellules pour obtenir 3 µg de protéines cellulaires et 250 µg de billes dans 50 µl. Les produits à tester (dilués dans 10% DMSO), en quantité de 10 µl à une concentration de 10 fois la concentration finale, sont distribués dans une plaque blanche 96 puits à fond clair (CORNING 3604 Polystyrene Non-Binding Surface). Le binding non-spécifique est défini par NDP-αMSH à 10⁻⁷ M. Le binding total est mesuré par le nombre de coups par minute en présence du radio-ligand seul. La distribution de la suspension membranes-billes (50 µl/puits) est suivie par la distribution de la solution de [¹²⁵I]-[Nle⁴, D-Phe⁷]-α-MSH, 40 µl/puits (100 pM concentration finale), pour un volume final de 100 µl/puits. Après 6 h. d'incubation à température ambiante, le comptage est fait dans un compteur de scintillation Microbeta TriLux. La valeur IC₅₀ des composés correspond à la concentration qui déplace la liaison spécifique du radio-ligand de 50%.

On détermine ainsi que les composés selon l'invention présentent une affinité .. pour les récepteurs MC3 et/ou MC4. Leurs IC₅₀ envers les récepteurs MC3 et MC4 sont inférieures à 10 µM, pour la plupart comprises entre 1 nM et 1 µM. A titre d'exemple, le composé n° 3 du tableau présente une IC₅₀ de 280 nM envers le récepteur MC4.

### Evaluation de l'activité agoniste des composés de formule (I) selon l'invention envers les récepteurs MC3 et MC4

Un test fonctionnel est utilisé pour discriminer l'activité agoniste de l'activité antagoniste. Pour cela, on dose la formation d'adénosine - mono-phosphate cyclique (AMPc) générée par l'activation du récepteur-MC3 ou du récepteur MC4.

Les cellules CHO-K1, exprimant le récepteur humain MC4 à une densité modérée (Euroscreen), sont ensemencées dans le milieu de culture DMEM / Nutrient Mix F12 (Gibco/BRI) contenant 10% de sérum de veau, 0.5% pyruvate de sodium, 1% L-glutamine, 1 % acides aminés non-essentiels, 200 mg/l hygromycine B et 0.5% PenStrep, ces produits étant fournis par Gibco/BRI, sauf le sérum de veau (Biowhittaker) et l'hygromycine B (Sigma).

Les cellules CHO(dhfr-) exprimant le récepteur humain MC3 sont ensemencées dans le milieu de culture MEM Eagle (Sigma) contenant 10% de sérum de veau dialysé, 1% L-glutamine, 1% pyruvate de sodium, 20mg/500 ml L-proline, 0.3 mg/ml Geneticin et 0.5% PenStrep, ces produits étant fournis par Gibco/BRI, sauf le sérum de veau dialysé (Cambrex) et la L-proline (Sigma).

Les composés à tester (dilués dans 10% DMSO), en quantité de 10 µl à une concentration de 10 fois la concentration finale, sont ajoutés aux plaques de cellules (volume final = 100 µl/puits). Après 1 heure d'incubation (37°C, 5% CO₂), la quantité du AMPc est dosée utilisant des kits du TROPIX (Appelera) selon la documentation du fournisseur. L'activité intrinsèque des composés est calculée en comparant la stimulation d'AMPc par ces composés à la stimulation induite par 30 nM de NDPαMSH (100% maximum). La valeur EC₅₀ des composés correspond à la concentration qui produit 50% de la stimulation maximale obtenue avec ce composé.

On détermine ainsi que les composés selon l'invention sont des agonistes des récepteurs MC3 et/ou MC4. Ils présentent des EC₅₀ envers les récepteurs MC3 et MC4 inférieures à 10 µM, pour la plupart comprises entre 1 nM et 1 µM. A titre d'exemples, le composé n° 3 du tableau présentent un EC₅₀ de 330 nM envers le récepteur MC3, et de 28 nM envers le récepteur MC4.

Les composés selon l'invention présentant une activité agoniste des récepteurs aux mélanocortines, ils peuvent donc être utilisés pour la préparation de médicaments. Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, dans les pathologies dans lesquelles les récepteurs aux mélanocortines, en particulier les récepteurs MC3 et/ou MC4, sont impliqués: il s'agit notamment du traitement et de la prévention de l'obésité, du diabète et des dysfonctions sexuelles pouvant affecter les deux sexes, telles que les dysfonctionnements érectiles, les maladies cardiovasculaires telles que les infarctus du myocarde ou l'hypertension ainsi que dans des applications antiinflammatoires ou dans le traitement de dépendance alcoolique.

Selon un autre de ses aspects, la présente invention concerne des compositions pharmaceutiques comprenant, en tant que principe actif, un composé selon l'invention: Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Une forme d'administration préférée est la voie orale.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I) : 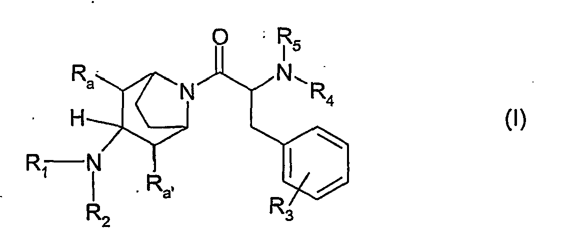 dans laquelle :
**Rₐ** et **R_{a'}**, identiques ou différents l'un de l'autre, représentent un atome d'hydrogène ou un groupe alkyle ou-cycloalkyle,
**R₁** représente un atome d'hydrogène ou un groupe alkyle, cycloalkyle, hétérocycloalkyle ou aryle,
**R₂** représente un groupe de formule -(CH₂)ₓ-(CO)_{y}-**Y** ou -(CO)_{y}-(CH₂)ₓ-**Y**, dans laquelle :
• x = 0, 1, 2, 3 ou 4,
• y = 0 ou 1,
• Y représente un atome d'hydrogène ou un groupe hydroxyle, alkyle, cycloalkyle, alcoxy, aryle, hétéroaryle ou -NR₁₁R₁₂, Y étant différent d'un atome d'hydrogène lorsque x=y= 0,
• R₁₁ et R₁₂, identiques ou différents l'un de l'autre, représentent un atome d'hydrogène ou un groupe alkyle, cycloalkyle, alcoxy ou -NR₁₃R₁₄, ou bien R₁₁ et R₁₂ forment ensemble, avec l'atome d'azote auquel ils sont rattachés, une structure mono-ou bicyclique comportant de 4 à 10 chaînons et comprenant éventuellement 1 à 3 hétéroatomes supplémentaires et/ou 1 à 3 insaturations éthyléniques ou acétyléniques, ce cycle étant éventuellement substitué en des positions quelconques par 1 à 3 groupes choisis parmi les atomes d'halogène et les groupes hydroxyles, alkyles, cycloalkyles, et alcoxy,
• R₁₃ et R₁₄, identiques ou différents l'un de l'autre, représentent un atome d'hydrogène ou un groupe alkyle, cycloalkyle ou alcoxy, ou bien R₁₃ et R₁₄ forment ensemble, avec l'atome d'azote auquel ils sont rattachés, une structure mono- ou bicyclique telle que définie ci-dessus,
**R₃** représente 1 à 3 groupes, identiques ou différents les uns des autres, situés en des positions quelconques du noyau auquel ils sont rattachés et choisis parmi les atomes d'halogène et les groupes alkyles, cycloalkyles, -OR, -NRR', -CO-NRR', -NR-COR', -NR-CO-NRR', -NR-COOR', -NO₂, -CN et -COOR,
**R₅** représente un atome d'hydrogène ou un groupe alkyle,
**R₄** est choisi parmi :
**(1)** un groupe de formule (a), (b) ou (c) éventuellement substitué par un groupe oxo : 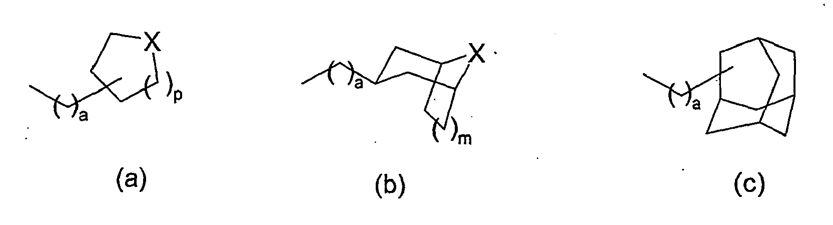 dans lesquelles chacun des cycles de formules (a), (b) et (c) peut être substitué, en des positions quelconques, par 1 à 4 groupes R₇, identiques ou différents les uns des autres, et dans lesquelles :
**a**=0, 1, 2 ou 3,
**p**=0, 1, 2 ou 3,
**m** = 0, 1 ou 2,
**X** représente un atome d'oxygène ou de soufre ou un chaînon -C(R₆)(R₇)- ou -N(R₁₀)-,
**R₆** est choisi parmi :
• un atome d'hydrogène, un atome d'halogène,
• un groupe -(CH₂)ₓ-OR₈, -(CH₂)ₓ-COOR₈, -(CH₂)ₓ-NR₈R₉, -(CH₂)ₓ-CO-NR₈R₉ ou -(CH₂)ₓ-NR₈-COR₉, dans lesquels x = 0, 1, 2, 3 ou 4,
• un groupe alkyle, cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle ou -CO-alkylhétéroaryle, , -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉,
• un groupe cycloalkyle ou hétérocycloalkyle fusionné ou non situé en position spiro sur le cycle de formule (a) auquel il est rattaché,
• un groupe cycloalkyle ou hétérocycloalkyle fusionné avec un groupe aryle ou hétéroaryle,
**R₇** est choisi parmi les atomes d'hydrogène et d'halogène et les groupes alkyles, cycloalkyles, aryles, hétéroaryles, alkylaryles, alkylhétéroaryles, -OR, -O-aryles, -O-hétéroaryles, -O-alkylaryles, -O-alkylhétéroaryles, -NRR', -CO-NRR', -NR-CO-R', -NR-CO-NRR', -NR-COOR', -NO₂, -CN et -COOR,
**R₈** et **R₉** sont choisis indépendamment l'un de l'autre parmi un atome d'hydrogène et les groupes alkyles, cycloalkyles, hétérocycloalkyles, aryles, hétéroaryles, alkylaryles, alkylhétéroaryles, -CO-alkyles, -CO-cycloalkyles, -CO-hétérocycloalkyles, -CO-aryles, -CO-hétéroaryles, -CO-alkylaryles, -CO-alkylhétéroaryles, -SO₂-alkyles, -SO₂-cycloalkyles, -SO₂-hétérocycloalkyle -SO₂-aryles, -SO₂-hétéroaryles, -SO₂-alkylaryles, -SO₂-alkylhétéroaryles, -C(=NH)-NRR', -COOR, -CO-NRR', -CS-NRR' et -(CH₂)ₓ-OR, où x = 0, 1, 2, 3 ou 4,
ou bien R₈ et R₉ forment ensemble un cycloalkyle ou un hétérocycloalkyle ;
**R₁₀** est choisi parmi :
• un atome d'hydrogène,
• un groupe -(CH₂)ₓ-OR₈, -(CH₂)ₓ-COOR₈, -(CH₂)ₓ-NR₈R₉, -(CH₂)ₓCO-NR₈R₉ ou -(CH₂)ₓ-NR₈-COR₉, -(CH₂)ₓ-COR₈ dans lesquels x = 0, 1, 2, 3 ou 4,
• un groupe cycloalkyle ou hétérocycloalkyle fusionné avec un groupe aryle ou hétéroaryles,
• un groupe alkyle, cycloalkyle, hétérocycloalkyle, aryle, hétéroaryle, alkylaryle, alkylhétéroaryle, -CO-alkyle, -CO-cycloalkyle, -CO-hétérocycloalkyle, -CO-aryle, -CO-hétéroaryle, -CO-alkylaryle, -CO-alkylhétéroaryle, -CS-alkyle, -CS-cycloalkyle, -CS-hétérocycloalkyle, -CS-aryle, -CS-hétéroaryle, -CS-alkylaryle, -CS-alkylhétéroaryle, -CS-NR₈R₉, -C(=NH)-NR₈R₉, -SO₂-alkyle, -SO₂-cycloalkyle, -SO₂-hétérocycloalkyle, -SO₂-aryle, -SO₂-hétéroaryle, -SO₂-alkylaryle, -SO₂-alkylhétéroaryle ou -SO₂-NR₈R₉,
• ou bien R₁₀ forme, avec l'atome d'azote auquel il est rattaché et un atome de carbone situé en une position quelconque de la structure cyclique de la formule (a), mais non adjacent audit atome d'azote, un pont comprenant de 3 à 5 chaînons,
**R** et **R'** représentent indépendamment l'un de l'autre un atome d'hydrogène ou un groupe alkyle, cycloalkyle, hétérocycloalkyle, aryle, hétéroaryle, alkylaryle ou alkylhétéroaryle,
les groupes alkyles, cycloalkyles, hétérocycloalkyles, aryles et hétéroaryles étant éventuellement substitués par un ou plusieurs groupes choisis parmi les groupes R, R', - OR, -NRR', -CO-NRR', -NR-CO-R', -NR-CO-NRR', -NO₂, -CN et -COOR, OCOR, COR, OCONRR', NRCOOR'
**(2)** un groupe de formule -A-R₁₈, -A-CH=N-R₁₉, -A-N(R₂₀)-A'-R₁₉, -A-CO-N(R₂₀)-A'-R₁₉, -A-CH(NH₂)-R₁₉ ou -A-N(R₂₀)-COO-A', dans lesquels A et A' représentent un groupe alkyle linéaire ou ramifié, R₁₈ représente un atome d'halogène ou un groupe -NH₂, hydroxyle ou phényle, R₁₉ représente un atome d'hydrogène ou un groupe hydroxyle, phényle, benzyle ou hétéroaryle, et R₂₀ représente un atome d'hydrogène ou un groupe benzyle,
**(3)** un groupe de formule (d) : 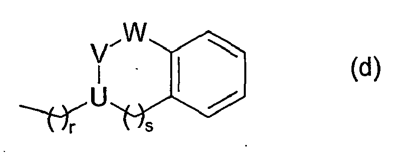 éventuellement substitué, en des positions quelconques, par 1 à 4 groupes R₇, identiques ou différents les uns des autres, tels que définis ci-dessus et dans laquelle r est égal à 1, 2 ou 3, s est égal à 0 ou 1, et l'un de U, V ou W représente un atome d'azote, les autres parmi U, V et W représentant des chaînons méthylène, ou
**(4)** un groupe -(CH₂)ᵣ-hétéroaryle, où r est égal à 1, 2 ou 3,
à l'état de base ou de sel d'addition à un acide, ainsi qu'à l'état d'hydrate ou de solvat.

2. Composé de formule (I) selon la revendication 1, **caractérisé en ce que** R₁ représente un groupe cycloalkyle,
à l'état de base ou de sel d'addition à un acide, ainsi qu'à l'état d'hydrate ou de solvat.

3. Composé de formule (I) selon la revendication 1 ou la revendication 2, **caractérisé en ce que** R₂ est choisi parmi les groupes suivants : -CO-R₁₅, -CO-NR₁₆R₁₇, -CO-NR₁₅NR₁₆R₁₇, -CO-aryle, -CO-hétéroaryle, -CO-(CH₂)ₓ-NR₁₆R₁₇, -(CH₂)ₓ-NR₁₆R₁₇, -(CH₂)ₓ-OH, -(CH₂)ₓ-aryle, -(CH₂)ₓ-hétéroaryle, -(CH₂)ₓ-CO-R₁₅ et -(CH₂)_{x'}-CO-NR₁₆R₁₇, dans lesquels :
• x = 0, 1, 2, 3 ou 4 et x' = 1, 2, 3 ou 4,
• R₁₅ représente un atome d'hydrogène ou un groupe alkyle, cycloalkyle ou alcoxy, et
• R₁₆ et R₁₇, identiques ou différents l'un de l'autre, représentent un atome d'hydrogène ou un groupe alkyle, cycloalkyle ou alcoxy, ou bien R₁₆ et R₁₇ forment ensemble, avec l'atome d'azote auquel ils sont rattachés, une structure mono- ou bicyclique comportant de 4 à 10 chaînons et comprenant éventuellement 1 à 3 hétéroatomes supplémentaires et/ou 1 à 3 insaturations éthyléniques ou acétyléniques, ce cycle étant éventuellement substitué en des positions quelconques par 1 à 3 groupes choisis parmi les atomes d'halogène et les groupes hydroxyles, alkyles, cycloalkyles et alcoxy,
à l'état de base ou de sel d'addition à un acide, ainsi qu'à l'état d'hydrate ou de solvat.

4. Composé de formule (I) selon l'une quelconque des revendications 1 à 3, **caractérisé en ce que** R₂ représente un groupe -CO-NR₁₆R₁₇, où R₁₆ et R₁₇ représentent des groupes alkyles ou alcoxy,
à l'état de base ou de sel d'addition à un acide, ainsi qu'à l'état d'hydrate ou de solvat.

5. Composé de formule (I) selon l'une quelconque des revendications 1 à 4, **caractérisé en ce que** R₃ représente 1 à 3 groupes, identiques ou différents les uns des autres, choisis parmi les atomes d'halogène,
à l'état de base ou de sel d'addition à un acide, ainsi qu'à l'état d'hydrate ou de solvat.

6. Composé de formule (I) selon l'une quelconque des revendications 1 à 5, **caractérisé en ce que** R₄ est choisi parmi :
(1) un groupe de formule (a-5), (a-6) ou (b-2) ci-dessous : 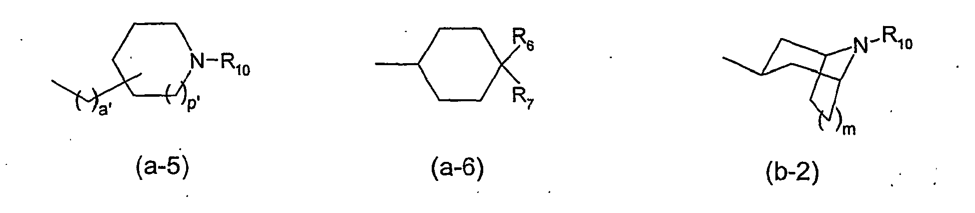 dans lesquelles chacun des cycles de formules (a-5), (a-6) et (b-2) peut être substitué, en des positions quelconques, par 1 à 4 groupes R₇, identiques ou différents les uns des autres, tels que définis dans la revendication 1, et dans lesquelles a' = 0 ou 1, p' = 0 ou 1 et R₆, R₇ et R₁₀ sont tels que définis dans la revendication 1,
(2) un groupe de formule -A-R₁₈ ou -A-CH=N-R₁₉, où A, R₁₈ et R₁₉ sont tels que définis dans la revendication 1,
(3) un groupe de formule (d-1), où r = 1, 2 ou 3 : 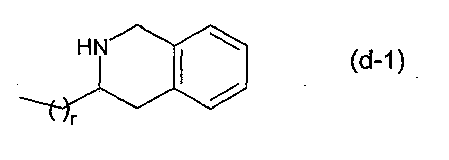
(4) un groupe -(CH₂)ᵣ-furyle ou -(CH₂)ᵣ-pyridinyle, où r est égal à 1, 2 ou 3,
à l'état de base ou de sel d'addition à un acide, ainsi qu'à l'état d'hydrate ou de solvat.

7. Composé de formule (I) selon l'une quelconque des revendications 1 à 6, **caractérisé en ce que** R₅ représente un atome d'hydrogène,
à l'état de base ou de sel d'addition à un acide, ainsi qu'à l'état d'hydrate ou de solvat.

8. Composé de formule (I) selon l'une quelconque des revendications 1 à 7, **caractérisé en ce que** Rₐ = R_{a'} = H,
à l'état de base ou de sel d'addition à un acide, ainsi qu'à l'état d'hydrate ou de solvat.

9. Procédé de préparation d'un composé de formule (I) selon l'une quelconque des revendications 1 à 8, **caractérisé en ce que** l'on réalise une amination réductrice d'un composé de formule (V) : 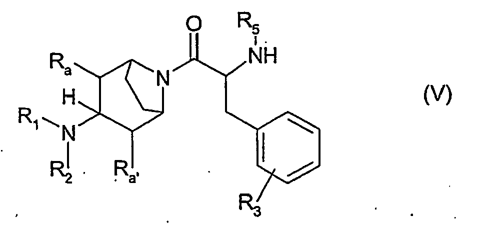 en présence d'un dérivé du groupe R₄ de type cétone, R₁₁ R₂, R₃, R₄, R₅, Rₐ et Rₐ, étant tels que définis dans l'une quelconque des revendications 1, à 8.

10. Composés de formules (IV) et (V), dans lesquelles R₁, R₂, R₃, R₅, Rₐ et Rₐ, sont tels que définis dans l'une quelconque des revendications 1, 2, 3, 4, 5, 7 et 8 et Pg représente un groupe protecteur:

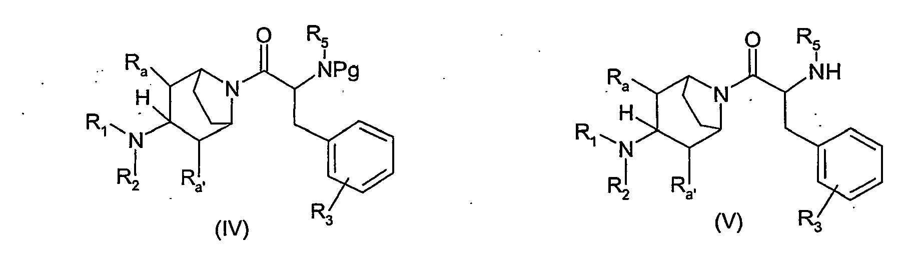

11. Médicament, **caractérisé en ce qu'**il comprend un composé de formule (I) selon l'une quelconque des revendications 1 à 8, ou un sel d'addition de ce composé à un acide pharmaceutiquement acceptable, ou encore un hydrate ou un solvat du composé de formule (I).

12. Composition pharmaceutique, **caractérisée en ce qu'**elle comprend un composé de formule (I) selon l'une quelconque des revendications 1 à 8, ou un sel pharmaceutiquement acceptable, un hydrate ou un solvat de ce composé, ainsi qu'au moins un excipient pharmaceutiquement acceptable.

13. Utilisation d'un composé de formule (I) selon l'une quelconque des revendications 1 à 8 pour la préparation d'un médicament destiné au traitement et à 1a prévention de l'obésité, du diabète et des dysfonctions sexuelles pouvant affecter les deux sexes au traitement des maladies cardiovasculaires ainsi que dans des applications anti-inflammatoires ou dans le traitement de dépendance alcoolique.

14. Utilisation selon la revendication 13, **caractérisée en ce que** lesdites dysfonctions sexuelles consistent en des dysfonctionnements érectiles.

15. Composés de formule (I) dont les noms suivent :
1 : *N*-[8-(4-chloro-*N*-piperidin-4-yl-D-phenylalanyl)-8-azabicyclo[3.2.1]oct-3-yl]-*N-*cyclohexyl-*N'*,*N'*-diethylurea
2: *N*-[8-(4-chloro-N-piperidin-3-yl-D-phenylalanyl)-8-azabicyclo[3.2.1]oct-3-yl]-*N-*cyclohexyl-*N'*,*N'*-diethylurea
3: *N*-{8-[*N*-(4-aminocyclohexyl)-4-chloro-D-phenylalanyl]-8-azabicyclo[3.2.1]oct-3-yl}-*N*-cyclohexyl-*N'*,*N'*-diethylurea
4: *N*-(8-[4-chloro-*N*-(tetrahydro-2*H*-pyran-4-y1)-D-phenylalanyl]-8-azabicyclo[3.2.1]oct-3-yl)-*N*-cyclohexyl-*N'*,*N'*-diethylurea
5: *N*-[8-(*N*-8-azabicyclo[3.2.1]oct-3-yl-4-chloro-D-phenylalanyl)-8-azabicyc1o[3.2.1]oct-3-yl]-*N*-cyclohexyl-*N'*,*N'*-diethylurea
6: *N*-{8-[4-chloro-*N*-(piperidin-4-ylmethyl)-D-phenylalanyl]-8-azabicyclo[3.2.1]oct-3-yl}-*N-*cyclohexyl-*N'*,*N'*-diethylurea
7: *N*-{8-[4-chloro-*N*-(piperidin-2-ylmethyl)-D-phenylalanyl]-8-azabicyclo[3.2.1]oct-3-yl}-*N*-cyclohexyl-*N'*-*N'*-diethylurea
8: *N*-{8-(4-chloro-*N*-(tetrahydro-3-thienyl)-D-phenylalanyl]-8-azabicyclo[3.2.1]oct-3-yl)-*N*-cyclohexyl-*N'*,*N'*-diethylurea
9: *N*-[8-(*N*-1-azabicyclo[2.2.2]oct-3-yl-4-chloro-D-phenylalanyl)-8-azabicyclo[3.2.1]oct-3-yl]-*N*-cyclohexyl-*N',N'*-diethylurea
10: *N*-[8-(*N*-azepan-4-yl-4-chloro-D-phenylalanyl)-8-azabicyclo[3.2.1]oct-3-yl]-*N* cyclohexyl-*N'*,*N'*-diethylurea
-11: *N*-[8-(4-chloro-*N*-{[(2*S*,4*R*)-4-hydroxypyrrolidin-2-yl]methyl}-D-phenylalanyl)-8-azabicyclo[3.2.1]oct-3-yl]-*N*-cyclohexyl-*N'*,*N'*-diethylurea
12: *N*-[8-(4-chloro-*N*-([(2R,4R)-4-hydroxypyrrolidin-2-yl]methyl)-D-phenylalanyl)-8-azabicyclo[3.2.1]oct-3-yl]-*N*-cyclohexyl-*N'*,*N'*-diethylurea
13: *N*-[8-(4-chloro- {[(2R,4S)-4-hydroxypyrrolidin-2-yl]methyl}-D-phenylalanyl)-8-azabicyclo[3.2.1]oct-3-yl]-*N*-cyclohexyl-*N'*,*N'*-diethylurea
14 : *N*-{8-[4-chloro-*N*-(1-phenylpiperidin-4-yl)-D-phenylalanyl]-8-azabicyclo[3.2.1]oct-3-yl}-*N*-cyclohexyl-*N*',*N*'-diethylurea
15: *N*-(8-{*N*-[(1-benzylpyrrolidin-3-yl)methyl]-4-chloro-D-phenylalanyl}-8-azabicyclo[3.2.1]oct-3-yl)-*N*-cyclohexyl-*N'*,*N'*-diethylurea
16: *N*-[8-(4-chloro-*N*-pyrrolidin-3-yl-D-phenylalanyl)-8-azabicyclo[3.2.1]oct-3-yl]-*N* cyclohexyl-*N'*,*N'*-diethylurea
17: *N*-(8-{4-chloro-*N*-[4-(4-hydroxyphenyl)cyclohexyl]-D-phenylalanyl}-8-azabicyclo[3.2.1]oct-3-yl)-*N*-cyclohexyl-*N'*,*N'*-diethylurea
18: *N*-{8-[*N*-(2-aminoethyl)-4-chloro-D-phenylalanyl]-8-azabicyclo[3.2.1]oct-3-yl)-*N*-cyclohexyl-*N'*,*N'*-diethylurea
19: *N*-{8-[*N*-(3-aminopropyl)-4-chloro-D-phenylalanyl]-8-azabicydo[3.2.1]oct-3-yl} *N*-cyclohexyl-*N*',*N*'-diethylurea
20: *N*'(8-{4-chloro-*N*-[(2E)-2-(hydroxyimino)-1-methylethyl]-D-phenylalanyl}-8-azabicyclo[3.2.1]oct-3-yl)-*N*-cyclohexyl-*N'*,*N'*-diethylurea
21: *N*-{8-[4-chloro-*N*-(2-fluoro-1-methylethyl)-D-phenylalanyl]-8-azabicyclo[3.2.1]oct-3-yl)-*N*-cyclohexyl-*N'*,*N'*-diethylurea
22: *N*-(8-{4-chloro-*N*-[(3R)-1,2,3,4-tetrahydroisoquinolin-3-ylmethyl]-D-phenylalanyl}-8-azabicyclo[3.2.1]oct-3-yl)-*N*-cyclohexyl-*N'*,*N*'-diethylurea
23: *N*-{8-[4-chloro-*N*-(pyridin-2-ylmethyl)-D-phenylalanyl]-8-azabicyclo[3.2.1]oct-3-yl}-*N*-cyclohexyl-*N*',*N*'-diethylurea
24: *N*-{8-[4-chloro-*N*-(2-furylmethyl)-D-phenylalanyl]-8-azabicyclo[3.2.1]oct-3-yl}-*N*-cyclohexyl-*N'*,*N'*-diethylurea
25: *N*-(8-{4-chloro-*N*-[(2R)-pyrrolidin-2-ylmethyl]-D-phenylalanyl}-8-azabicyclo[3.2.1]oct-3-yl)-*N*-cyclohexyl-*N'*,*N'*-diethylurea
26: *N*-(8-{4-chloro-*N*-[(2S)-pyrrolidin-2-ylmethyl]-D-phenylalanyl}-8-azabicyclo[3.2.1]oct-3-yl)-*N*-cyclohexyl-*N'*,*N'*-diethylurea
27: *N*-[8-(*N*-azetidin-3-yl-4-chloro-D-phenylalanyl)-8-azabicyclo[3.2.1]oct-3-yl]-*N-*cyclohexyl-*N'*,*N'*-diethylurea
28: *N*-(8-{*N*-[(1-benzylpyrrolidin-3-yl)methyl]-4-chloro-D-phenylalanyl)-8-azabicyclo[3.2.1]oct-3-yl)-*N*-cyclohexyl-*N'*,*N'*-diethylurea

## Claims

1. Compound corresponding to formula (I): 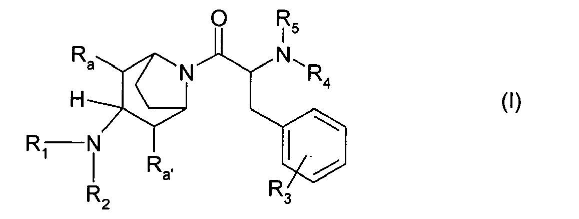 in which:
**Rₐ** and **R_{a'}**, which may be identical to or different from one another, represent a hydrogen atom, or an alkyl or cycloalkyl group,
**R₁** represents a hydrogen atom, or an alkyl, cycloalkyl, heterocycloalkyl or aryl group,
**R₂** represents a group of formula -(CH₂)ₓ-(CO)y-**Y** or -(CO)_{y}-(CH₂)ₓ-**Y**, in which:
• x = 0, 1, 2, 3 or 4,
• y = 0 or 1,
• **Y** represents a hydrogen atom, or a hydroxyl, alkyl, cycloalkyl, alkoxy, aryl, heteroaryl or -NR₁₁R₁₂ group, Y being different from a hydrogen atom when x = y = 0,
• R₁₁ and R₁₂ which may be identical to or different from one another, represent a hydrogen atom, or an alkyl, cycloalkyl, alkoxy or -NR₁₃R₁₄ group, or else R₁₁ and R₁₂ form, together with the nitrogen atom to which they are attached, a mono- or bicyclic structure containing from 4 to 10 ring members and optionally comprising 1 to 3 additional hetero atoms and/or 1 to 3 ethylenic or acetylenic unsaturations, this ring being optionally substituted in any of the positions with 1 to 3 groups chosen from halogen atoms, and hydroxyl, alkyl, cycloalkyl and alkoxy groups,
• R₁₃ and R₁₄, which may be identical to or different from one another, represent a hydrogen atom, or an alkyl, cycloalkyl or alkoxy group, or else R₁₃ and R₁₄ form, together with a nitrogen atom to which they are attached, a mono- or bicyclic structure as defined above,
**R₃** represents 1 to 3 groups, which may be identical to or different from one another, located in any positions of the ring to which they are attached and chosen from halogen atoms, and alkyl, cycloalkyl, -OR, -NRR', -CO-NRR', -NR-CO-R', -NR-CO-NRR', -NR-COOR', -NO₂, -CN and -COOR groups,
**R₅** represents a hydrogen atom or an alkyl,
**R₄** is chosen from:
**(1)** a group of formula (a), (b) or (c) optionally substituted with an oxo group: 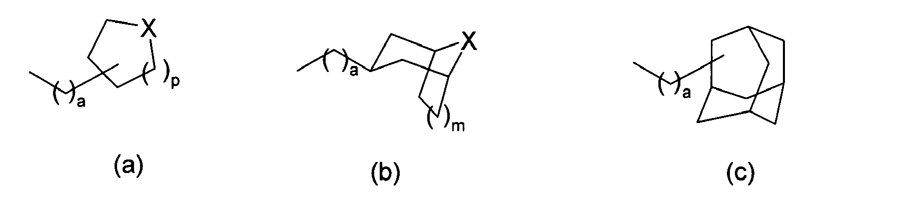 in which each of the rings of formulae (a), (b) and (c) may be substituted, in any positions, with 1 to 4 groups R₇, which may be identical to or different from one another, and in which:
**a** = 0, 1, 2 or 3,
**p** = 0, 1, 2 or 3,
**m** = 0, 1 or 2,
**X** represents an oxygen or sulphur atom, or a ring member -C (R₆) (R₇) - or -N(R₁₀)-,
**R₆** is chosen from:
• a hydrogen atom, a halogen atom,
• a group -(CH₂)ₓ-OR₈, - (CH₂)ₓ-COOR₈, - (CH₂)ₓ-NR₈R₉, -(CH₂)ₓ-CO-NR₈R₉ or - (CH₂)ₓ-NR₈-COR₉, in which x = 0, 1, 2, 3 or 4,
• an alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -CS-alkyl, -CS-cycloalkyl, -CS-heterocycloalkyl, -CS-aryl, -CS-heteroaryl, -CS-alkylaryl, -CS-alkylheteroaryl, -CS-NR₈R₉ or -C(=NH) -NR₈R₉ group,
• a fused or nonfused cycloalkyl or heterocycloalkyl group located in the spiro position on the ring of formula (a) to which it is attached,
. a cycloalkyl or heterocycloalkyl group fused with an aryl or heteroaryl group,
**R₇** is chosen from hydrogen and halogen atoms, and alkyl, cycloalkyl, aryl, heteroaryl, alkylaryl, alkylheteroaryl, -OR, -O-aryl, -0-heteroaryl, -0-alkylaryl, -O-alkylheteroaryl, -NRR', -CO-NRR', -NR-CO-R', -NR-CO-NRR', -NR-COOR', -NO₂, -CN and -COOR groups,
**R₈** and **R₉** are chosen, independently of one another, from a hydrogen atom, and alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl, -C(=NH)-NRR', -COOR, -CO-NRR', -CS-NRR' and - (CH₂)ₓ-OR groups, where x = 0, 1, 2, 3 or 4;
or else R₈ and R₉ together form a cycloalkyl or a heterocycloalkyl;
**R₁₀** is chosen from:
• a hydrogen atom,
• a group - (CH₂) X-OR₈, - (CH₂)ₓ-COOR₈, -(CH₂)ₓ-NR₈R₉, - ( CH₂)ₓ-CO-NR₈R₉, - (CH₂)ₓ-NR₈-COR₉ or - (CH₂)ₓ-COR₈, in which x = 0, 1, 2, 3 or 4,
• a cycloalkyl or heterocycloalkyl group fused with an aryl or heteroaryl group,
• ain alkyl, cycloalkyl, heterocycloalkyl, aryl, heteroaryl, alkylaryl, alkylheteroaryl, -CO-alkyl, -CO-cycloalkyl, -CO-heterocycloalkyl, -CO-aryl, -CO-heteroaryl, -CO-alkylaryl, -CO-alkylheteroaryl, -CS-alkyl, -CS-cycloalkyl, -CS-heterocycloalkyl, -CS-aryl, -CS-heteroaryl, -CS-alkylaryl, -CS-alkylheteroaryl, -CS-NR₈R₉, -C (=NH) -NR₈R₉, -SO₂-alkyl, -SO₂-cycloalkyl, -SO₂-heterocycloalkyl, -SO₂-aryl, -SO₂-heteroaryl, -SO₂-alkylaryl, -SO₂-alkylheteroaryl or -SO₂-NR₈R₉ group,
• or else R₁₀ forms, with the nitrogen atom to which it is attached and a carbon atom located in any position of the cyclic structure of formula (a), but not adjacent to said nitrogen atom, a bridge comprising from 3 to 5 members,
**R** and **R'** represent, independently of one another, a hydrogen atom, or an alkyl, cycloalkyl, heterocycloalkyl, aryl, heteroaryl, alkylaryl or alkylheteroaryl group,
the alkyl, cycloalkyl, heterocycloalkyl, aryl and heteroaryl groups being optionally substituted with one or more groups chosen from the groups R, R', -OR, -NRR', -CO-NRR', -NR-CO-R', -NR-CO-NRR', -NO₂, -CN and -COOR, OCOR, COR, OCONRR', NRCOOR',
**(2)** a group of formula -A-R₁₈, -A-CH=N-R₁₉, -A-N (R₂₀) -A' -R₁₉, -A-CO-N (R₂₀) -A' -R₁₉, -A-CH (NH₂) -R₁₉ or -A-N (R₂₀) -COO-A', in which A and A' represent a linear or branched alkyl group, R₁₈ represents a halogen atom, or an -NH₂, hydroxyl or phenyl group, R₁₉ represents a hydrogen atom, or a hydroxyl, phenyl, benzyl or heteroaryl group, and R₂₀ represents a hydrogen atom or a benzyl group,
**(3)** a group of formula (d): 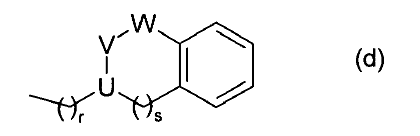 optionally substituted, in any positions, with 1 to 4 groups R₇, which may be identical to or different from one another, as defined above, and in which r is equal to 1, 2 or 3, s is equal to 0 or 1, and one of U, V or W represents a nitrogen atom, the others among U, V and W representing methylene ring members, or
**(4)** a -(CH₂)ᵣ-heteroaryl group, where r is equal to 1, 2 or 3,
in the form of a base or of an addition salt with an acid, and also in the form of a hydrate or of a solvate.

2. Compound of formula (I) according to Claim 1, **characterized in that** R₁ represents a cycloalkyl group,
in the form of a base or of an addition salt with an acid, and also in the form of a hydrate or of a solvate.

3. Compound of formula (I) according to Claim 1 or Claim 2, **characterized in that** R₂ is chosen from the following groups: -CO-R₁₅, -CO-NR₁₆R₁₇, -CO-NR₁₅-NR₁₆R₁₇, -CO-aryl, -CO-heteroaryl, -CO- (CH₂)ₓ-NR₁₆R₁₇, - (CH₂)ₓ-NR₁₆R_{17'} - (CH₂)ₓ-OH, - (CH₂)ₓ-aryl, -(CH₂)ₓ-heteroaryl, -(CH₂)_{x'}-CO-R₁₅ and - (CH₂)_{x'} -CO-NR₁₆R₁₇, in which:
• x = 0, 1, 2, 3 or 4 and x' = 1, 2, 3 or 4,
• R₁₅ represents a hydrogen atom, or an alkyl, cycloalkyl or alkoxy group, and
R₁₆ and R₁₇, which may be identical to or different from one another, represent a hydrogen atom, or an alkyl, cycloalkyl or alkoxy group, or else R₁₆ and R₁₇ form, together with the nitrogen atom to which they are attached, a mono- or bicyclic structure containing from 4 to 10 ring members and optionally comprising 1 to 3 additional hetero atoms and/or 1 to 3 ethylenic or acetylenic unsaturations, this ring being optionally substituted in any positions with 1 to 3 groups chosen from halogen atoms, and hydroxyl, alkyl, cycloalkyl and alkoxy groups,
in the form of a base or of an addition salt with an acid, and also in the form of a hydrate or of a solvate.

4. Compound of formula (I) according to any one of Claims 1 to 3, **characterized in that** R₂ represents a group -CO-NR₁₆R₁₇, where R₁₆ and R₁₇ represent alkyl or alkoxy groups,
in the form of a base or of an addition salt with an acid, and also in the form of a hydrate or of a solvate.

5. Compound of formula (I) according to any one of Claims 1 to 4, **characterized in that** R₃ represents 1 to 3 groups, which may be identical to or different from one another, chosen from halogen atoms,
in the form of a base or of an addition salt with an acid, and also in the form of a hydrate or of a solvate.

6. Compound of formula (I) according to any one of Claims 1 to 5, **characterized in that** R₄ is chosen from:
(1) a group of formula (a-5), (a-6) or (b-2) below: 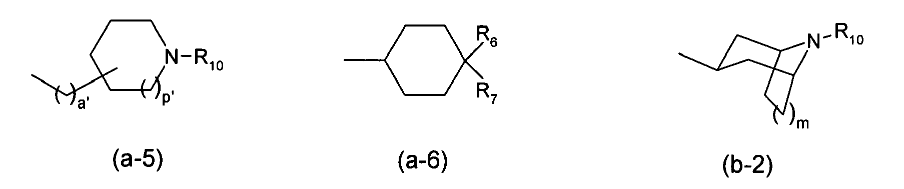 in which each of the rings of formulae (a-5), (a-6) and (b-2) can be substituted, in any positions, with 1 to 4 groups R₇, which may be identical to or different from one another, as defined in Claim 1, and in which a' = 0 or 1, p' = 0 or 1, and R₆, R₇ and R₁₀ are as defined in Claim 1,
(2) a group of formula -A-R₁₈ or -A-CH=N-R₁₉, where A, R₁₈ and R₁₉ are as defined in Claim 1,
(3) a group of formula (d-1), where r = 1, 2 or 3: 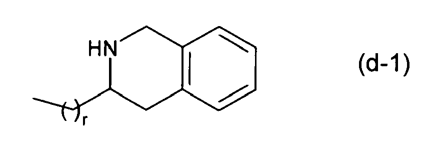
(4) a group -(CH₂)ᵣ-furyl or -(CH₂)ᵣ-pyridinyl, where r is equal to 1, 2 or 3,
in the form of a base or of an addition salt with an acid, and also in the form of a hydrate or of a solvate.

7. Compound of formula (I) according to any one of Claims 1 to 6, **characterized in that** R₅ represents a hydrogen atom,
in the form of a base or of an addition salt with an acid, and also in the form of a hydrate or of a solvate.

8. Compound of formula (I) according to any one of Claims 1 to 7, **characterized in that** Rₐ = R_{a'} = H,
in the form of a base or of an addition salt with an acid, and also in the form of a hydrate or of a solvate.

9. Method for preparing a compound of formula (I) according to any one of Claims 1 to 8, **characterized in that** a reductive amination of a compound of formula (V): 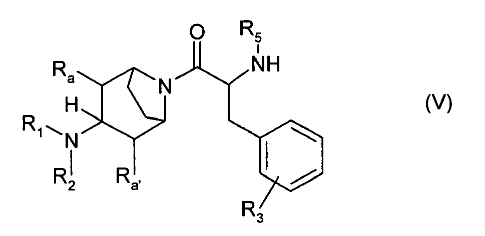 is carried out in the presence of a derivative of the group R₄ of ketone type, R₁, R₂, R₃, R₄, R₅, Rₐ and R_{a'} being as defined in any one of Claims 1 to 8.

10. Compounds of formulae (IV) and (V), in which R₁, R₂, R₃, R₅, Rₐ and R_{a'} are as defined in any one of Claims 1, 2, 3, 4, 5, 7 and 8, and Pg represents a protective group:

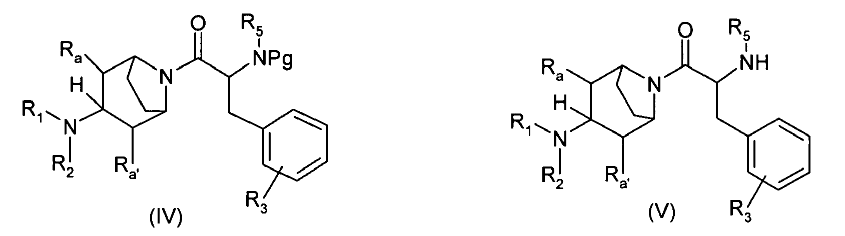

11. Medicament, **characterized in that** it comprises a compound of formula (I) according to any one of Claims 1 to 8, or an addition salt of this compound with a pharmaceutically acceptable acid, or else a hydrate or a solvate of the compound of formula (I).

12. Pharmaceutical composition, **characterized in that** it comprises a compound of formula (I) according to any one of Claims 1 to 8, or a pharmaceutically acceptable salt, a hydrate or a solvate of this compound, and also at least one pharmaceutically acceptable excipient.

13. Use of a compound of formula (I) according to any one of Claims 1 to 8, in the manufacture of a medicament for use in the treatment and in the prevention of obesity, diabetes and sexual dysfunctions that can affect both sexes, in the treatment of cardiovascular diseases, and also in antiinflammatory uses or in the treatment of alcohol dependency.

14. Use according to Claim 13, **characterized in that** said sexual dysfunctions consist of erectile dysfunctions.

15. Compounds of formula (I) having the following names:
1: *N*-[8-(4-chloro-*N*-piperidin-4-yl-D-phenylalanyl)-8-azabicyclo[3.2.1]oct-3-yl]-*N*-cyclohexyl-*N'*,*N'*-diethylurea
2: *N*-[8-(4-chloro-*N*-piperidin-3-yl-D-phenylalanyl)-8-azabicyclo[3.2.1]oct-3-yl]-*N*-cyclohexyl-*N*',*N*'-diethylurea
3: *N*-{8-[*N*-(4-aminocyclohexyl)-4-chloro-D-phenylalanyl]-8-azabicyclo[3.2.1]oct-3-yl}-*N*-cyclohexyl-*N'*,*N*'-diethylurea
4: *N*-{8-[4-chloro-*N*-(tetrahydro-2H-pyran-4-yl)-D-phenylalanyl]-8-azabicyclo[3.2.1]oct-3-yl}-*N-*cyclohexyl-*N'*,*N'*-diethylurea
5: *N*-[8-(*N*-8-azabicyclo[3.2.1]oct-3-yl-4-chloro-D-phenylalanyl)-8-azabicyclo[3.2.1]oct-3-yl]-N-cyclohexyl-*N'*,*N'*-diethylurea
6: *N*-{8-[4-chloro-*N*-(piperidin-4-ylmethyl)-D-phenylalanyl]-8-azabicyclo[3.2.1]oct-3-yl}-*N*-cyclohexyl-*N*',*N*'-diethylurea
7: *N*-{8-[4-chloro-*N*-(piperidin-2-ylmethyl)-D-phenylalanyl]-8-azabicyclo[3.2.1]oct-3-yl}-*N*-cyclohexyl-*N'*,*N'*-diethylurea
8: *N*-{8-[4-chloro-*N*-(tetrahydro-3-thienyl)-D-phenylalanyl]-8-azabicyclo[3.2.1]oct-3-yl}-*N*-cyclohexyl-*N'*,*N'*-diethylurea
9: *N*-[8-(*N*-1-azabicyclo[2.2.2]oct-3-yl-4-chloro-D-phenylalanyl)-8-azabicyclo[3.2.1]oct-3-yl]-*N-*cyclohexyl-*N'*,*N'*-diethylurea
10: *N*-[8-(*N*-azepan-4-yl-4-chloro-D-phenylalanyl)-8-azabicyclo[3.2.1]oct-3-yl]-*N*-cyclohexyl-*N'*,*N'*-diethylurea
11: *N*- [8-(4-chloro-*N*-{[(2S, 4*R*)-4-hydroxy-pyrrolidin-2-yl]methyl}-D-phenylalanyl)-8-aza-bicyclo[3.2.1]oct-3-yl]-*N*-cyclohexyl-*N'*,*N'*-diethylurea
12 : *N*-[8-(4-chloro-*N*-{[(2R, 4R)-4-hydroxy-pyrrolidin-2-yl]methyl}-D-phenylalanyl)-8-aza-bicyclo[3.2.1]oct-3-yl]-*N*-cyclohexyl-*N'*,*N'*-diethylurea
13: *N*-[8-(4-chloro-*N*-{[(2*R*, 4*S*)-4-hydroxy-pyrrolidin-2-yl]methyl}-D-phenylalanyl)-8-aza-bicyclo[3.2.1]oct-3-yl]-*N*-cyclohexyl-*N'*,*N'*-diethylurea
14: *N*-{8-[4-chloro-*N*-(1-phenylpiperidin-4-yl)-D-phenylalanyl]-8-azabicyclo[3.2.1]oct-3-yl}-*N-*cyclohexyl-*N'*,*N'*-diethylurea
15: *N*-(8-{*N*-[(1-benzylpyrrolidin-3-yl)-methyl]-4-chloro-D-phenylalanyl}-8-azabicyclo-[3.2.1]oct-3-yl)-*N*-cyclohexyl-*N'*,*N'*-diethylurea
16: *N*-[8-(4-chloro-*N*-pyrrolidin-3-yl-D-phenylalanyl)-8-azabicyclo[3.2.1]oct-3-yl]-*N*-cyclohexyl-*N'*,*N'*-diethylurea
17 : *N*-(8-{4-chloro-*N*-[4-(4-hydroxyphenyl)-cyclohexyl]-D-phenylalanyl}-8-azabicyclo[3.2.1]oct-3-yl)-*N*-cyclohexyl-*N'*,*N'*-diethylurea
18: *N*-{8-[*N*-(2-aminoethyl)-4-chloro-D-phenylalanyl]-8-azabicyclo[3.2.1]oct-3-yl}-*N*-cyclohexyl-*N', N'*-diethylurea
19: *N*-{8-[*N*-(3-aminopropyl)-4-chloro-D-phenylalanyl]-8-azabicyclo[3.2.1]oct-3-yl}-N-cyclohexyl-*N'*,*N'*-diethylurea
20: *N*-(8-{4-chloro-*N*-[(2E)-2-(hydroxyimino)-1-methylethyl]-D-phenylalanyl}-8-azabicyclo[3.2.1]oct-3-yl)-*N*-cyclohexyl-*N'*,*N'*-diethylurea
21: *N*-{8-[4-chloro-*N*-(2-fluoro-1-methylethyl)-D-phenylalanyl]-8-azabicyclo[3.2.1]oct-3-yl}-N-cyclohexyl-*N'*,*N'*-diethylurea
22: *N*-(8-{4-chloro-*N*-[(3R)-1,2,3,4-tetra-hydroisoquinolin-3-ylmethyl]-D-phenylalanyl}-8-azabicyclo[3.2.1]oct-3-yl)*-N*-cyclohexyl-*N'*,*N'*-diethylurea
23: *N*-{8-[4-chloro-*N*-(pyridin-2-ylmethyl)-D-phenylalanyl]-8-azabicyclo[3.2.1]oct-3-yl}-*N*-cyclohexyl-*N'*,*N'*-diethylurea
24: *N*-{8-[4-chloro-*N*-(2-furylmethyl)-D-phenylalanyl]-8-azabicyclo[3.2.1]oct-3-yl}-*N*-cyclohexyl-*N'*,*N'*-diethylurea
25: *N*-(8-{4-chloro-*N*-[(2*R*)-pyrrolidin-2-yl-methyl]-D-phenylalanyl}-8-azabicyclo[3.2.1]oct-3-yl)-*N-*cyclohexyl-*N'*,*N'*-diethylurea
26: *N*-(8-{4-chloro-*N*-[(2*S*)-pyrrolidin-2-yl-methyl]-D-phenylalanyl}-8-azabicyclo[3.2.1]oct-3-yl)-*N-*cyclohexyl-*N'*,*N'*-diethylurea
27: *N*-[8-(iN-azetidin-3-yl-4-chloro-D-phenylalanyl)-8-azabicyclo[3.2.1]oct-3-yl]-*N*-cyclohexyl-*N'*,*N'*-diethylurea
28: *N*-(8-{*N*-[(1-benzylpyrrolidin-3-yl)-methyl]-4-chloro-D-phenylalanyl}-8-azabicyclo[3.2.1]-oct-3-yl)-*N*-cyclohexyl-*N'*,*N'*-diethylurea

## Patentansprüche

1. Verbindung der Formel (I), 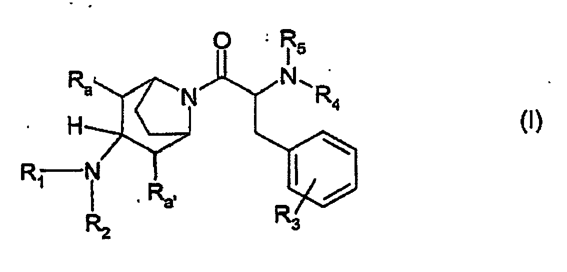 mit den folgenden Bedeutungen:
**Rₐ** und **R_{a'}** sind gleich oder verschieden voneinander und bedeuten ein Wasserstoffatom oder eine Alkyl- oder Cycloalkylgruppe,
**R₁** bedeutet ein Wasserstoffatom oder eine Alkyl-, Cycloalkyl-, Heterocycloalkyl- oder Arylgruppe,
**R₂** bedeutet eine Gruppe der Formel -(CH₂)ₓ-(CO)_{y}-**Y** oder -(CO)_{y}-(CH₂)ₓ-**Y**, mit den folgenden Bedeutungen:
. x = 0, 1, 2, 3 oder 4,
. y = 0 oder 1,
. **Y** bedeutet ein Wasserstoffatom oder eine Hydroxy-, Alkyl-, Cycloalkyl-, Alkoxy-, Aryl-, Heteroaryl- oder -NR₁₁R₁₂-Gruppe, wobei, wenn x = y = 0, Y nicht ein Wasserstoffatom bedeutet,
. R₁₁ und R₁₂ sind gleich oder voneinander verschieden und bedeuten ein Wasserstoffatom oder eine Alkyl-, Cycloalkyl-, Alkoxy- oder -NR₁₃R₁₄-Gruppe, oder R₁₁ und R₁₂ bilden gemeinsam mit dem Stickstoffatom, an das sie gebunden sind, eine mono- oder bicyclische Struktur mit 4 bis 10 Kettengliedern und gegebenenfalls mit 1 bis 3 zusätzlichen Heteroatomen und/oder 1 bis 3 Ethylen- oder Acetylen-Ungesättigtheiten, wobei dieser Cyclus gegebenenfalls in beliebigen Stellungen durch 1 bis 3 Gruppen aus der Reihe Halogenatome und Hydroxy-, Alkyl-, Cycloalkyl- und Alkoxygruppen substituiert ist,
. R₁₃ und R₁₄ sind gleich oder verschieden voneinander und bedeuten ein Wasserstoffatom oder eine Alkyl-, Cycloalkyl- oder Alkoxygruppe, oder R₁₃ und R₁₄ bilden gemeinsam mit dem Stickstoffatom, an das sie gebunden sind, eine mono- oder bicyclische Struktur wie oben definiert,
**R₃** bedeutet 1 bis 3 Gruppen, die gleich oder verschieden voneinander sind und die in beliebigen Stellungen des Rings, an den sie gebunden sind, vorliegen und aus der Reihe Halogenatome und Alkyl-, Cycloalkyl-, -OR-, -NRR'-, -CO-NRR'-, -NR-CO-R'-, -NR-CO-NRR'-, NR-COOR'-, -NO₂-, -CN- und COOR-Gruppen stammen,
**R₅** bedeutet ein Wasserstoffatom oder eine Alkylgruppe,
**R₄** stammt aus der Reihe:
**(1)** Gruppe der Formel (a), (b) oder (c), die gegebenenfalls durch eine Oxo-Gruppe substituiert ist, 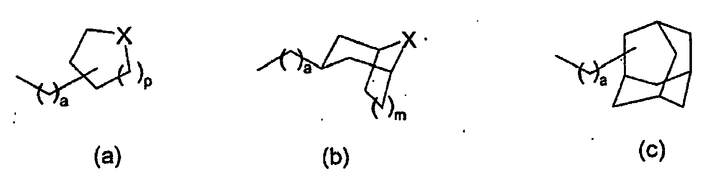 in denen jeder der Ringe der Formeln (a), (b) und (c) gegebenenfalls in beliebigen Stellungen durch 1 bis 4 R₇-Gruppen, die gleich oder voneinander verschieden sind, substituiert sein kann, mit den folgenden Bedeutungen:
**a** = 0, 1, 2 oder 3,
**p** = 0, 1, 2 oder 3,
**m** = 0, 1 oder 2,
**X** bedeutet ein Sauerstoff- oder Schwefelatom oder ein Ringglied -C(R₆) (R₇)- oder -N (R₁₀) -,
**R₆** stammt aus der Reihe:
. Wasserstoffatom, Halogenatom,
. -(CH₂)ₓ-OR₈-, -(CH₂)ₓ-COOR₈-, -(CH₂)ₓ-NR₈R₉-, - (CH2)ₓ-CO-NR₈R₉- oder - (CH₂)ₓ-NR₈-COR₈-Gruppe,
wobei x = 0, 1, 2, 3 oder 4,
. eine Alkyl-, Cycloalkyl-, Heterocycloalkyl-Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl- oder -CO-Alkylheteroaryl-, -CS-Alkyl-, -CS-Cycloalkyl-, -CS-Heterocycloalkyl-, -CS-Aryl-, -CS-Heteroaryl-, -CS-Alkylaryl-, -CS-Alkylheteroaryl-, -CS-NR₈-R₉-, -C(=NH)-NR₈R₉-Gruppe,
. eine Cycloalkyl- oder Heterocycloalkylgruppe, die gegebenenfalls in spiro-Stellung an dem Ring der Formel (a), an den sie gebunden ist, anelliert ist,
. eine Cycloalkyl- oder Heterocycloalkylgruppe, die mit einer Aryl- oder Heteroarylgruppe anelliert ist,
**R₇** stammt aus der Reihe Wasserstoff- und Halogenatome und Alkyl-, Cycloalkyl-, Aryl-, Heteroaryl-, Alkylaryl-, Alkylheteroaryl-, -OR-, -0-Aryl-, -O-Heteroaryl-, -0-Alkylaryl-, -O-Alkylheteroaryl-, -NRR'-, -CO-NRR'-, -NR-CO-R'-, -NR-CO-NRR'-, -NR-COOR'-, -NO₂-, -CN- und COOR-Gruppen,
**R₈** und **R₉** stammen unabhängig voneinander aus der Reihe Wasserstoffatom und Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl-, - C (=NH) -NRR' -, -COOR-, -CO-NRR' -, -CS-NRR' - und -(CH₂)ₓ-OR- Gruppen, wobei x = 0, 1, 2, 3 oder 4, oder R₈ und R₉ bilden gemeinsam ein Cycloalkyl oder ein Heterocycloalkyl;
**R₁₀** stammt aus der Reihe:
. Wassertoffatom,
. - (CH₂)ₓ-OR₈-, - (CH₂)ₓ-COOR₈-, - (CH₂)ₓ-NR₈R₉-, - (CH₂)ₓ-CO-NR₈R₉- oder - (CH₂)ₓ -NR₈-COR₉-, - (CH₂)ₓ COR₈-Gruppe, wobei x = 0, 1, 2, 3 oder 4,
. Cycloalkyl- oder Heterocycloalkylgruppe, die mit einer Aryl- oder Heteroarylgruppe anelliert ist,
. Alkyl-, Cycloalkyl-, Heterocycloalkyl-, Aryl-, Heteroaryl-, Alkylaryl-, Alkylheteroaryl-, -CO-Alkyl-, -CO-Cycloalkyl-, -CO-Heterocycloalkyl-, -CO-Aryl-, -CO-Heteroaryl-, -CO-Alkylaryl-, -CO-Alkylheteroaryl-, -CS-Alkyl-, -CS-Cycloalkyl-, -CS-Heterocycloalkyl-, -CS-Aryl-, -CS-Heteroaryl-, -CS-Alkylaryl-, -CS-Alkylheteroaryl-, -CS-NR₈R₉-, -C (=NH) -NR₈R₉-, -SO₂-Alkyl-, -SO₂-Cycloalkyl-, -SO₂-Heterocycloalkyl-, -SO₂-Aryl-, -SO₂-Heteroaryl-, -SO₂-Alkylaryl-, -SO₂-Alkylheteroaryl- oder -SO₂-NR₈R₉-Gruppe,
. oder R₁₀ bildet gemeinsam mit dem Stickstoffatom, an das es gebunden ist, und einem Kohlenstoffatom in einer beliebigen Stellung der Ringstruktur der Formel (a), jedoch nicht benachbart zu dem genannten Stickstoffatom, eine Brücke mit 3 bis 5 Brückengliedern,
**R** und **R'** bedeuten unabhängig voneinander ein Wasserstoffatom oder eine Alkyl-, Cycloalkyl-, Heterocycloalkyl-, Aryl-, Heteroaryl-, Alkylaryl- oder Alkylheteroarylgruppe,
wobei die Alkyl-, Cycloalkyl-, Heterocycloalkyl-, Aryl- und Heteroarylgruppen gegebenenfalls durch eine oder mehrere Gruppen aus der Reihe R, R', -OR, -NRR', -CO-NRR', -NR-CO-R', -NR-CO-NRR', -NO₂, -CN und -COOR, OCOR, COR, OCONRR', NRCOOR' substituiert sind,
**(2)** Gruppe der Formel -A-R₁₈, -A-CH=N-R₁₉, -AN (R₂₀) -A' -R₁₉, -A-CO-N (R₂₀) -A' -R₁₉, -A-CH(NH₂)-R₁₉ oder -AN(R₂₀)-COO-A', mit den folgenden Bedeutungen: A und A' bedeuten eine geradkettige oder verzweigte Alkylgruppe, R₁₈ bedeutet ein Halogenatom oder eine -NH₂-, Hydroxy-oder Phenylgruppe, R₁₉ bedeutet ein Wasserstoffatom oder eine Hydroxy-, Phenyl-, Benzyl- oder Heteroarylgruppe, und R₂₀ bedeutet ein Wasserstoffatom oder eine Benzylgruppe,
**(3)** Gruppe der Formel (d), 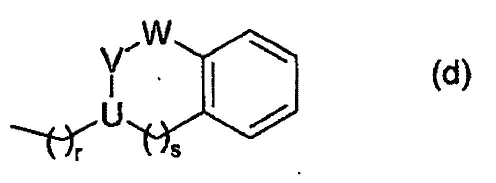 die gegebenenfalls in beliebigen Stellungen durch 1 bis 4 R₇-Gruppen, die gleich oder voneinander verschieden sind, wie oben definiert, substituiert sind und in der r 1, 2 oder 3 bedeutet, s 0 oder 1 bedeutet und eines der Symbole U, V oder W ein Stickstoffatom bedeutet, während die andere Symbole aus der Reihe U, V und W Methylenglieder bedeuten, oder
**(4)** -(CH₂)ᵣ-Heteroarylgruppe, wobei r 1, 2 oder 3 bedeutet,
als Base oder als Säureadditionssalz, sowie als Hydrat oder als Solvat.

2. Verbindung der Formel (I) nach Anspruch 1, **dadurch gekennzeichnet, daß** R₁ eine Cycloalkylgruppe bedeutet, als Base oder als Säureadditionssalz, sowie als Hydrat oder als Solvat.

3. Verbindung der Formel (I) nach Anspruch 1 oder Anspruch 2, **dadurch gekennzeichnet, daß** R₂ aus der Reihe der folgenden Gruppen stammt: -CO-R₁₅, -CO-NR₁₆R₁₇, -CO-NR₁₅-NR₁₆R₁₇, -CO-Aryl, -CO-Heteroaryl, -CO-(CH₂)ₓ-NR₁₆R₁₇, - (CH₂)ₓ-NR₁₆R₁₇, - (CH₂)ₓ-OH, - (CH₂) ₓ-Aryl, - (CH₂)ₓ-Heteroaryl, -(CH₂)ₓ-CO-R₁₅ und - (CH₂) ₓ-CO-NR₁₆R₁₇ mit den folgenden Bedeutungen:
. x = 0, 1, 2, 3 oder 4 und x' = 1, 2, 3 oder 4,
. R₁₅ bedeutet ein Wasserstoffatom oder eine Alkyl-, Cycloalkyl- oder Alkoxygruppe, und
. R₁₆ und R₁₇ sind gleich oder voneinander verschieden und bedeuten ein Wasserstoffatom oder eine Alkyl-, Cycloalkyl- oder Alkoxygruppe, oder R₁₆ und R₁₇ bilden gemeinsam mit dem Stickstoffatom, an das sie gebunden sind, eine mono- oder bicyclische Struktur mit 4 bis 10 Kettengliedern und gegebenenfalls mit 1 bis 3 zusätzlichen Heteroatomen und/oder 1 bis 3 Ethylen-oder Acetylen-Ungesättigtheiten, wobei dieser Cyclus gegebenenfalls in beliebigen Stellungen durch 1 bis 3 Gruppen aus der Reihe Halogenatome und Hydroxy-, Alkyl-, Cycloalkyl- und Alkoxygruppen substituiert ist, als Base oder als Säureadditionssalz, sowie als Hydrat oder als Solvat.

4. Verbindung der Formel (I) nach einem der Ansprüche 1 bis 3, **dadurch gekennzeichnet, daß** R₂ eine -CO-NR₁₆R₁₇-Gruppe bedeutet oder R₁₆ und R₁₇ Alkyl- oder Alkoxygruppen bedeuten, als Base oder als Säureadditionssalz, sowie als Hydrat oder als Solvat.

5. Verbindung der Formel (I) nach einem der Ansprüche 1 bis 4, **dadurch gekennzeichnet, daß** R₃ 1 bis 3 gleiche oder voneinander verschiedene Gruppen aus der Reihe der Halogenatome bedeutet, als Base oder als Säureadditionssalz, sowie als Hydrat oder als Solvat.

6. Verbindung der Formel (I) nach einem der Ansprüche 1 bis 5, **dadurch gekennzeichnet, daß** R₄ aus der folgenden Reihe stammt:
(1) Gruppe der Formel (a-5), (a-6) oder (b-2) unten: 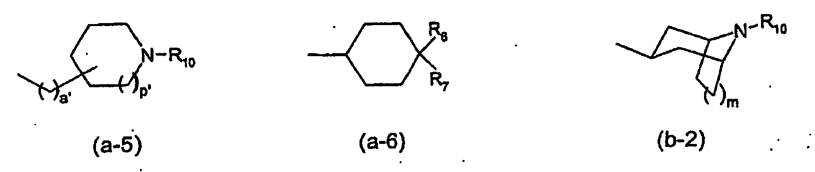 in denen jeder der Ringe der Formeln (a-5), (a-6) und (b-2) in beliebigen Stellungen durch 1 bis 4 gleiche oder voneinander verschiedene Gruppen R₇ wie in Anspruch 1 definiert substituiert sein können, und in denen a' = 0 oder 1, p' = 0 oder 1 und R₆, R₇ und R₁₀ wie in Anspruch 1 definiert sind,
(2) Gruppe der Formel -A-R₁₈ oder -A-CH=N-R₁₉, wobei A, R₁₈ und R₁₉ wie in Anspruch 1 definiert sind,
(3) Gruppe der Formel (d-1), wobei r = 1, 2 oder 3, 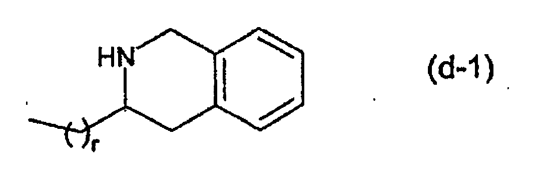
(4) -(CH₂)ᵣ-Furylgruppe oder - (CH₂)ᵣ-Pyridinylgruppe, wobei r 1, 2 oder 3 bedeutet, als Base oder als Säureadditionssalz, sowie als Hydrat oder als Solvat.

7. Verbindung der Formel (I) nach einem der Ansprüche 1 bis 6, **dadurch gekennzeichnet, daß** R₅ ein Wasserstoffatom bedeutet, als Base oder als Säureadditionssalz, sowie als Hydrat oder als Solvat.

8. Verbindung der Formel (I) nach einem der Ansprüche 1 bis 7, **dadurch gekennzeichnet, daß** Rₐ = R_{a'} = H bedeutet,
als Base oder als Säureadditionssalz, sowie als Hydrat oder als Solvat.

9. Verfahren zur Herstellung einer Verbindung der Formel (I) nach einem der Ansprüche 1 bis 8, **dadurch gekennzeichnet, daß** man eine Verbindung der Formel (V) 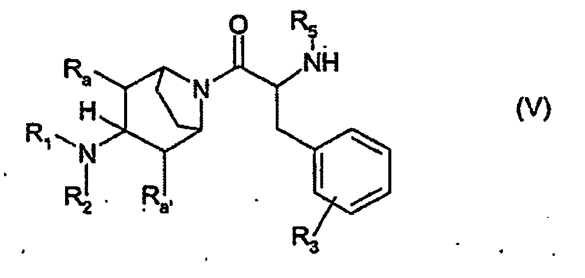 in Gegenwart eines Derivats der R₄-Gruppe des Ketontyps reduktiv aminiert, wobei R₁, R₂, R₃, R₄, R₅, Rₐ und R_{a'} wie in einem der Ansprüche 1 bis 8 definiert sind.

10. Verbindungen der Formeln (IV) und (V), in denen R₁, R₂, R₃, R₅, Rₐ und R_{a'} wie in einem der Ansprüche 1, 2, 3, 4, 5, 7 und 8 definiert sind und Pg eine Schutzgruppe bedeutet:

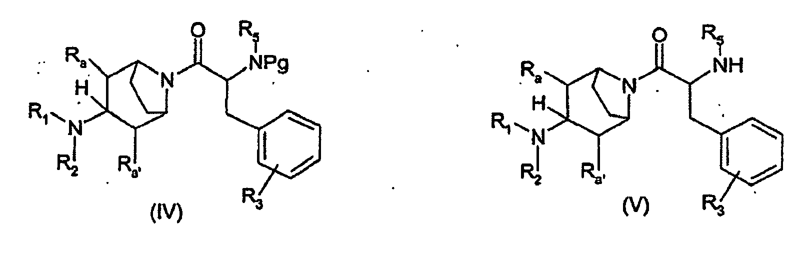

11. Arzneimittel, **dadurch gekennzeichnet, daß** es eine Verbindung der Formel (I) nach einem der Ansprüche 1 bis 8 oder ein Additionssalz dieser Verbindung mit einer pharmazeutisch unbedenklichen Säure oder ein Hydrat oder ein Solvat der Verbindung der Formel (I) enthält.

12. Pharmazeutische Zusammensetzung, **dadurch gekennzeichnet, daß** sie eine Verbindung der Formel (I) nach einem der Ansprüche 1 bis 8 oder ein Additionssalz dieser Verbindung mit einer pharmazeutisch unbedenklichen Säure oder ein Hydrat oder ein Solvat der Verbindung der Formel (I) sowie mindestens ein pharmazeutisch unbedenkliches Bindemittel enthält.

13. Verwendung einer Verbindung der Formel (I) nach einem der Ansprüche 1 bis 8 für die Herstellung eines Arzneimittels zur Behandlung und Vorbeugung von Fettleibigkeit, Diabetes und Sexualdysfunktionen, die beide Geschlechter betreffen können, für die Behandlung von Herz-Kreislauf-Krankheiten sowie bei entzündungshemmenden Anwendungen oder bei der Behandlung von Alkoholismus.

14. Verwendung nach Anspruch 13, **dadurch gekennzeichnet, daß** die Sexualdysfunktionen aus erektilen Dysfunktionen bestehen.

15. Verbindungen der Formel (I) mit den folgenden Bezeichnungen:
1: *N*-[8-(4-Chlor-*N*-piperidin-4-yl-D-phenylalanyl)-8-azabicyclo[3.2.1]oct-3-yl]-*N*-cyclohexyl-*N*',*N*'-diethylharnstoff
2: *N*-[8-(4-Chlor-*N*-piperidin-3-yl-D-phenylalanyl)-8-azabicyclo[3.2.1]oct-3-yl]-*N*-cyclohexyl-*N*',*N*'-diethylharnstoff
3: *N*-{8-[*N*-(4-Aminocyclohexyl)-4-chlor-D-phenylalanyl]-8-azabicyclo[3.2.1]oct-3-yl}-*N-*cyclohexyl-*N'*,*N'*-diethylharnstoff
4: *N*-{8-[4-Chlor-*N*-(tetrahydro-2H-pyran-4-yl)-D-phenylalanyl]-8-azabicyclo[3.2.1]oct-3-yl}-*N-*cyclohexyl-*N'*,*N'*-diethylharnstoff
5: *N*-[8-(*N*-8-azabicyclo[3.2.1]oct-3-yl-4-chlor-D-phenylalanyl)-8-azabicyclo[3.2.1]oct-3-yl]-*N-*cyclohexyl-*N'*,*N'*-diethylharnstoff
6: *N*-{8-[4-Chlor-*N*-(piperidin-4-ylmethyl)-D-phenylalanyl]-8-azabicyclo[3.2.1]oct-3-yl}-*N-*cyclohexyl-*N'*,*N'*-diethylharnstoff
7: *N*-{8-[4-Chlor-*N*-(piperidin-2-ylmethyl)-D-phenylalanyl]-8-azabicyclo[3.2.1]oct-3-yl}-N-cyclohexyl-*N'*,*N'*-diethylharnstoff
8: *N*-{8-[4-Chlor-*N*-(tetrahydro-3-thienyl)-D-phenylalanyl]-8-azabicyclo[3.2.1]oct-3-yl}-N-cyclohexyl-*N'*,*N'*-diethylharnstoff
9: *N*-[8-(*N*-1-azabicyclo[2.2.2]oct-3-yl-4-chlor-D-phenylalanyl)-8-azabicyclo[3.2.1]oct-3-yl]-N-cyclohexyl-*N'*,*N'*-diethylharnstoff
10: *N*-[8-(*N*-Azepan-4-yl-4-chlor-D-phenylalanyl)-8-azabicyclo[3.2.1]oct-3-yl]-*N*-cyclohexyl-*N'*,*N'-*diethylharnstoff
11: *N*-[8-(4-Chlor-*N*-{[(2*S*, 4*R*)-4-hydroxypyrrolidin-2-yl]methyl}-D-phenylalanyl)-8-azabicyclo[3.2.1]oct-3-yl]-*N*-cyclohexyl-*N'*,*N'*-diethylharnstoff
12: *N*-[8-(4-Chlor-*N*-{[(2*R*,4*R*)-4-hydroxypyrrolidin-2-yl]methyl}-D-phenylalanyl)-8-azabicyclo[3.2.1]oct-3-yl]-*N*-cyclohexyl-*N'*,*N'*-diethylharnstoff
13: *N*-[8-(4-Chlor-*N*-{[(2*R*,4*S*)-4-hydroxypyrrolidin-2-yl]methyl}-D-phenylalanyl)-8-azabicyclo[3.2.1]oct-3-yl]-*N*-cyclohexyl-*N'*,*N'*-diethylharnstoff
14: *N*-{8-[4-Chlor-*N*-(1'-phenylpiperidin-4-yl)-D-phenylalanyl]-8-azabicyclo[3.2.1]oct-3-yl}-N-cyclohexyl-*N'*,*N'*-diethylharnstoff
15: *N*-(8-{*N*-[(1-Benzylpyrrolidin-3-yl)methyl]-4-chlor-D-phenylalanyl}-8-azabicyclo[3.2.1]oct-3-yl}-*N-*cyclohexyl-*N'*,*N'*-diethylharnstoff
16: *N*-[8-(4-Chlor-*N*-pyrrolidin-3-yl-D-phenylalanyl)-8-azabicyclo[3.2.1]oct-3-yl]-*N*-cyclohexyl-*N'*,*N'-*diethylharnstoff
17: *N*-[8-{4-Chlor-*N*-[4-(4-hydroxyphenyl)cyclohexyl]-D-phenylalanyl}-8-azabicyclo[3.2.1]oct-3-yl]-*N-*cyclohexyl-*N'*,*N'*-diethylharnstoff
18: *N*-{8-[*N*-(2-Aminoethyl)-4-chlor-D-phenylalanyl]-8-azabicyclo[3.2.1]oct-3-yl}-*N*-cyclohexyl-*N'*,*N'-*diethylharnstoff
19: *N*-{8-[*N*-(3-Aminopropyl)-4-chlor-D-phenylalanyl]-8-azabicyclo[3.2.1]oct-3-yl}-*N*-cyclohexyl-*N'*,*N'-*diethylharnstoff
20: *N*-(8-{4-Chlor-*N*-[(2*E*)-2-(hydroxyimino)-1-methylethyl]-D-phenylalanyl}-8-azabicyclo[3.2.1]oct-3-yl)-*N*-cyclohexyl-*N'*,*N'*-diethylharnstoff
21: *N*-{8-[4-Chlor-*N*-(2-fluor-1-methylethyl)-D-phenylalanyl]-8-azabicyclo[3.2.1]oct-3-yl}-*N-*cyclohexyl-*N'*,*N'*-diethylharnstoff
22: *N*-(8-{4-Chlor-*N*-[(3R)-1,2,3,4-tetrahydroisochinolin-3-ylmethyl]-D-phenylalanyl}-8-azabicyclo[3.2.1]oct-3-yl)-*N*-cyclohexyl-*N'*,*N'-*diethylharnstoff
23: *N*-{8-[4-Chlor-*N*-(pyridin-2-ylmethyl)-D-phenylalanyl]-8-azabicyclo[3.2.1]oct-3-yl}-N-cyclohexyl-*N'*,*N'*-diethylharnstoff
24: *N*-{8-[4-Chlor-*N*-(2-furylmethyl)-D-phenylalanyl]-8-azabicyclo[3.2.1]oct-3-yl}-*N*-cyclohexyl-*N'*,*N'-*diethylharnstoff
25: *N*-(8-{4-Chlor-*N*-[(2R)-pyrrolidin-2-ylmethyl]-D-phenylalanyl}-8-azabicyclo[3.2.1]oct-3-yl)-*N-*cyclohexyl-*N'*,*N'*-diethylharnstoff
26: *N*-(8-{4-Chlor-*N*-[(2S)-pyrrolidin-2-ylmethyl]-D-phenylalanyl}-8-azabicyclo[3.2.1]oct-3-yl)-*N-*cyclohexyl-*N'*,*N'*-diethylharnstoff
27: *N*-[8-(*N*-Azetidin-3-yl-4-chlor-D-phenylalanyl)-8-azabicyclo[3.2.1]oct-3-yl]-*N*-cyclohexyl-*N'*,*N'-*diethylharnstoff
28: *N*-(8-{*N*-[(1-Benzylpyrrolidin-3-yl)methyl]-4-chlor-D-phenylalanyl}-8-azabicyclo[3.2.1]oct-3-yl)-*N-*cyclohexyl-*N'*,*N'*-diethylharnstoff
